# EUROPEAN PATENT APPLICATION

(11) **EP 3 388 419 A1**
(43) Date of publication of application: **17.10.2018**
(21) Application number: 17166194.5
(22) Date of filing: 12.04.2017
(51) Int. Cl.: C07D 215/28, C07D 498/04, A61K 38/00, A61K 31/47

(54) **GLI1 INHIBITORS AND USES THEREOF**

(71) Applicant: Leadiant Biosciences SA, 6850 Mendrisio (CH)
(72) Inventor: GIANNINI, Giuseppe, 00071 Pomezia (ROMA) (IT); TADDEI, Maurizio, 53035 Monteriggioni (SI) (IT); MANETTI, Fabrizio, 53019 Castelnuovo Berardenga (SI) (IT); PETRICCI, Elena, 53100 Siena (IT); STECCA, Barbara, 50141 Firenze (IT)
(74) Representative: Ghirardi, Valeria

(57) **Abstract**

The present invention relates to compounds able to inhibit the protein Gli1 having the formula 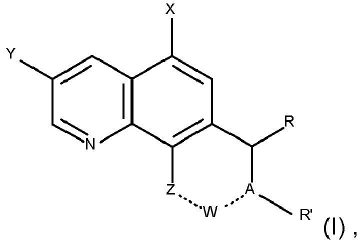 wherein the meanings of the substituents are indicated in the description, for use in the treatment of diseases related to Gli-1. The present invention also relates to compounds of formula (IIa) and (IIb) and to their use as medicaments, in particular for the treatment of diseases related to Gli1, more in particular for the treatment of tumors. Pharmaceutical compositions comprising said compounds of formula (IIa) or (IIb) are also within the scope of the present invention.

## Description

### FIELD OF THE INVENTION

The present invention relates to compounds able to inhibit the protein Gli1.

In particular, it relates to compounds for use for Gli1 inhibition.

The present invention also relates to newly synthesized compounds and to their use as medicaments, in particular for the treatment of diseases related to Gli1, more in particular for the treatment of tumors.

### BACKGROUND OF THE INVENTION

Glioma-associated oncogene homologue 1 (Gli1) is a zinc finger transcription factor with a key well documented role in oncogenesis (Dahmane N. 1997 Nature 389:876-881. Nilsson M 2000 PNAS 97:3438-3443. Grachtchouk M. 2000 Nat. Genet. 24:216-217. Kimura H 2005 Oncogene 24:4026-4036. Sheng H 2002 Cancer Res. 62:5308-5316.). Gli1 is the downstream effector of the Hedgehog (HH) signaling pathway. Nevertheless, the most recent literature suggests that Gli proteins can be directly or indirectly modulated by many different oncogenic and proliferative inputs even independently from upstream HH signaling.

The activation of the canonical HH signaling pathway is mediated by HH proteins that bind the transmembrane receptor PATCHED (PTCH), relieving its inhibitory effect on SMOOTHENED (SMO), which moves at the tip of the cilium. Consequently, active SMO triggers a cascade of events leading to the activation of the GLI transcription factors and their translocation inside the nucleus (Tang C.M. 2016 Oncotarget Oct 25. D'Amico D. 2016 Trends Mol. Med. 22: 851-862.). Once inside the nucleus, Gli1 binds the DNA by its zinc-finger domain activating the transcription of different genes coding for proteins and growth factors involved in tumorigenesis, angiogenesis and metastasis.

Canonical and non-canonical hyperactivation of Gli1 occurs in a variety of human cancers, including medulloblastoma, skin, brain, lungs, prostate, gastrointestinal tract and blood cancers.

Therefore, it is strongly desirable to inhibit the Gli1 proteins.

SMO inhibition is considered a good strategy for Gli1 inhibition. However, the anticancer activity of HH inhibitors targeting SMO is strictly dependent on their ability to inhibit HH downstream effector Gli1.

Two SMO antagonists Vismodegib (Proctor A.E. 2014 Ann. Pharmacother. 48:99-106) and Sonidegib (Collier N.J. 2016 Expert Rev. Anticancer Ther. 16: 1011-1018), have recently been approved by the FDA for Basal Cell Carcinoma (BCC) treatment, and clinical trials for colorectal, pancreatic, small-cell lung cancers, chondrosarchoma as well as medulloblastoma are actually ongoing (Sekulic A. 2016 Cell 164: 831).

However, after few years from their approval, tumor resistance has been observed as a main problem in patients treated with these drugs.

It is also known that Gli activation occurs by the interplay between HH-Gli and PI3K/AKT or RAS/RAF/MEK and other oncogenic signaling pathways (i.e.WIP-1, ERK, PKA, MAPK, Bcl-2, etc). These crosstalks, able to generate HH independent Gli1 activation, have been recently reviewed by many authors, and lead to non-canonical HH pathway activation (Pandolfi S. 2015 Expert Rev. Mol. Med. 17:e5. Stecca B. 2010 J. Mol. Cell Biol. 2: 84-95).

It is therefore evident that an efficient anticancer therapy for HH dependent tumors and any tumor related to an abnormal Gli1 activation can not be limited to an inhibition upstream of Gli1.

The Gli1 activation by many non-canonical pathways can be considered as one of causes responsible for tumor resistance observed, for example, in patients treated with HH antagonists (Vlčková K. 2016 Int. J. Oncol. 49: 953-960. Arnhold V 2016 Cancer Chemother. Pharmacol. 77: 495-505.).

In this scenario, the development of molecules able to directly target Gli1, the final effector of the HH and other oncogenic pathways, represents a very innovative and suitable approach to cancer therapy (Di Magno L. 2015 Biochimica Biophysica Acta 1856: 62-72.).

Therefore, compounds able to directly inhibit the protein Gli1 are desired.

Despite the many Smo antagonists reported in the literature, present in clinical trials or already approved by the FDA for cancer treatment, only few inhibitors of Gli1 are known so far (Rimkus T. K. 2016 Cancers 8, 22.).

GANT-61 and Zerumbone are the reference compounds used to inhibit Gli1 (and Gli2) in biological studies (Lauth M. 2007 PNAS 104: 8455-8460; Geng L et al, 2016 Oncotarget doi: 10.18632/oncotarget.9792).

With their very symmetric and almost flat structure (see scheme 1 below) as well as poor solubility, these molecules can not be considered good starting points to obtain drug candidates.

More recently a natural product, Glabrescione-B, has been reported as a Gli1 inhibitor (Infante P. 2015 EMBO J. 34: 200-216.); this product can not be considered as well a good drug candidate for its structure and properties, such as a high hydrophobicity and metabolic stability.

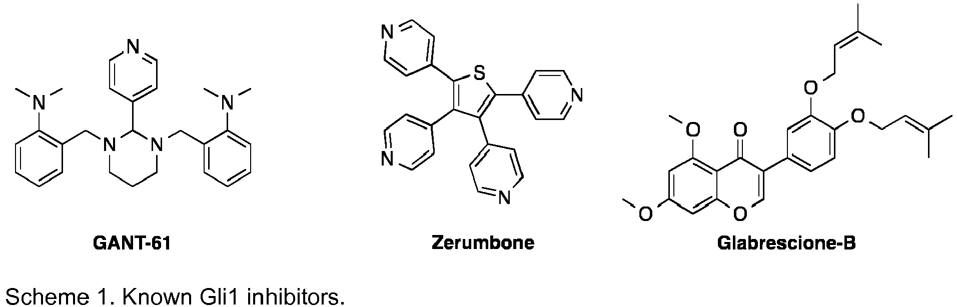

Therefore, it would be particularly desirable to have small molecule compounds able to target and selectively inhibit Gli1.

In particular, compounds able to specifically target the Gli1 protein can be advantageous in avoiding the onset of tumor resistance, observed, for example, in the treatment with SMO antagonists.

It has now been found a class of compounds able to efficiently inhibit Gli1.

Such compounds can be particularly effective in targeting cancer stem cell populations, since growing evidence suggests that cancer stem cells in several types of cancer require active Gli1 for their survival and self-renewal. For instance, pharmacological and genetic inhibition of GLI1 drastically reduces self-renewal *in vitro* and growth *in vivo* of ALDH^{high} melanoma stem cells (Santini R, 2012 Stem Cells 30:1808-18); Inhibition of Gli1/2 with CK2a reduces stem-like side population of human lung cancer cells (Zhang S 2012, Plos One 7:e38996); Targeting Gli1 suppresses cell growth and enhances chemosensitivity in CD34+ acute myeloid leukemia progenitor cells (Long B 2016 Cell Physiol Biochem 38:1288-302); GANT61 decreases CSC population of triple-negative breast cancers (TNBC), which have a very poor prognosis. Interestingly, combination treatment of paclitaxel and GANT61 shows additive effects in reducing CSCs (Koike Y 2017 Breast Cancer). Therefore, compounds able to specifically inhibit Gli1 can be efficiently used in cancers, in particular specifically with the aim of reducing cancer stem cells.

In addition, the GLI1 inhibitors of the invention can be advantageously used for increasing the effects of chemotherapeutic drugs. Indeed, there is evidence in the literature that the combined treatment of a Gli1 inhibitor and a chemotherapeutic agent is particularly effective in the treatment of cancer (see for example the following papers: i) Graab U et al, 2015. Identification of a novel synthetic lethality of combined inhibition of hedgehog and PI3K signaling in rhabdomyosarcoma. Oncotarget 6:8722; *ii)* Vlčková K, et al, 2016. GLI inhibitor GANT61 kills melanoma cells and acts in synergy with obatoclax. Int J Oncol 49:953; *iii)* Miyazaki Y et al, 2016. Efficient elimination of pancreatic cancer stem cells by hedgehog/GLI inhibitor GANT61 in combination with mTOR inhibition. Mol Cancer 15:49; *iv)* Kurebayashi J, et al, 2017. Anti-cancer stem cell activity of a hedgehog inhibitor GANT61 in estrogen receptor-positive breast cancer cells. Cancer Sci. doi: 10.1111/cas.13205; *v)* Li J et al, 2016. GANT61, a GLI inhibitor, sensitizes glioma cells to the temozolomide treatment. J Exp Clin Cancer Res; 35:184; *vi)* Lin Z et al, 2016. Suppression of GLI sensitizes medulloblastoma cells to mitochondria-mediated apoptosis. J Cancer Res Clin Oncol 142:2469; *vii)* Gonnissen A et al, 2017. The effect of metformin and GANT61 combinations on the radiosensitivity of prostate cancer cells. Int J Mol Sci;18(2); *viii)* Kern D et al, 2015. Hedgehog/GLI and PI3K signaling in the initiation and maintenance of chronic lymphocytic leukemia. Oncogene 34: 5341).

Therefore, the compounds of the invention thanks to their inhibition activity of the Gli1 protein can also be advantageously used in combination with a chemotherapeutic agent, in particular for providing a synergic effect on the reduction of tumor growth.

In particular, Gli1 inhibitors of the present invention are characterized by a 3,4-dihydro-*2H-*[1,3]oxazino[5,6-h]quinoline nucleus variously decorated in different positions.

Thanks to their activity on Gli1 such compounds can be used in the treatment of diseases related to the overexpression of Gli1 and/or to the HH pathway.

Some of the compounds for the use of the invention are already known in the field but for very different applications.

For example, WO2011106226 discloses their use for inhibiting hypoxia inducible factor (HIF) for protection from hypoxic damage; WO2011022721 discloses using similar compounds for inhibiting botulinum neurotoxic activity, WO2009137597 for the treatment of neurodegenerative diseases, WO2008024922 for treatment of metalloproteinases-related diseases, WO2008014602 for treatment of age-related disorders. WO2011082175 discloses the use of compounds of this class as chondroitin sulfate inhibitors.

Some of these compounds are also disclosed in scientific publications for other uses, such as anti-microbics (De La Fuente, R. et al. Small molecules with antimicrobial activity against E. coli and P. aeruginosa identified by high-throughput screening. Br. J. Pharmacol. 2006, 149(5), 551-559) for inhibition of Botulinum toxin (Caglic, D. et al. Identification of clinically viable quinolinol inhibitors of botulinum neurotoxin A light chain. J.Med. Chem. 2014, 57 (3), 669-676) or for inhibition of HIV (Serrao, E. et al. Fragment-based discovery of 8-hydroxyquinoline inhibitors of the HIV-1 integrase-lens epithelium-derived growth factor/p75 (IN-LEDGF/p75) interaction. J. Med. Chem. 2013, 56(6), 2311-2322).

Some compounds have also been found to induce apoptosis (Chung, K.-S. et al. Identification of small molecules inducing apoptosis by cell-based assay using fission yeast deletion mutants. Invest. New Drugs 2008, 26 (4), 299-307).

Some documents disclose the use of some compounds of this class for the treatment of tumors related to the Wnt signaling pathway. For example, WO201014948 and the work "Modulation of Wnt/β-catenin signaling and proliferation by a ferrous iron chelator with therapeutic efficacy in genetically engineered mouse models of cancer, Coombs, G. S. et al, Oncogene 2012, 31(2), 213-225), disclose the use of such compounds as Wnt inhibitor compounds for the treatment of tumors mediated by a Wnt signaling pathway.

However, no mention is made in none of these prior-art documents to the inhibition of Gli1 or to the treatment of diseases involving an alteration of Gli1 or related to the HH pathway.

The inhibitory effect of Gli1 provides the compounds of the invention with the advantage of being particularly effective in tumors mediated by the HH signaling pathway and in tumors wherein Gli1, an important therapeutic target, is overexpressed.

### SUMMARY OF THE INVENTION

It is an object of the present invention a compound characterized by the following formula (I) 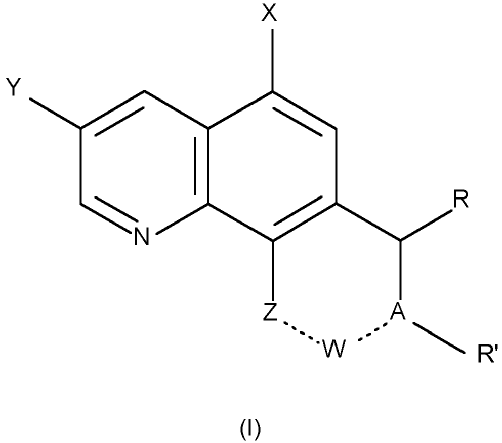 wherein
A is selected from N and NH,
X is selected from H and a halogen;
Y is selected from the group consisting of H, halogen, O-C₁-C₈alkyl, linear or branched, O-C₅-C₁₂aryl, O-C₁-C₈acyl, linear or branched, and O-C₃-C₈cycloalkyl;
Z is selected from O and OH;
W is CH₂ or CO or is absent and when W is CH₂ or CO each dashed line indicates a single bond,
R and R' are independently selected from the group consisting of: H; C₁-C₈alkyl, linear or branched; C₁-C₈acyl, linear or branched; C₃-C₈cycloalkyl or heterocycloalkyl; C₅-C₁₄aryl or heteroaryl; CH(COOCH₃)(CH₂phenyl) and C₁-C₈alkylene-R²,
   wherein R² is selected from the group consisting of H, C₁-C₈alkyl, linear or branched, C₁-C₈acyl, linear or branched, C₃-C₈cycloalkyl or heterocycloalkyl, C₅-C₁₄aryl or heteroaryl,
said heterocycloalkyl and heteroaryl containing one or more heteroatoms selected from N, S and O in a monocyclic or polycyclic system,
said cycloalkyl, heterocycloalkyl, aryl or heteroaryl being optionally substituted on any position of the ring with one or more substituents selected from the group consisting of halogen, C₁-C₄alkyl, linear or branched, O-C₁-C₄alkyl, linear or branched, C₁-C₈acyl, linear or branched, an amino group optionally substituted with one or two C₁-C₄alkyl groups, SH and OH,
the enantiomers, diastereoisomers, and mixtures thereof,
or a pharmaceutically acceptable salt, hydrate or solvate thereof,
for use in the treatment of a disease which can be improved or prevented by the inhibition of the Gli1 protein.

Preferably, said disease is a tumor or metastasis, in particular a Hedgehog dependent tumor. More preferably, said tumor is selected from the group consisting of basal cell carcinoma, medulloblastoma, skin tumor, brain tumor, lungs tumor, prostate tumor, breast tumor, gastrointestinal tract tumor, blood tumors, and melanoma.

In a preferred embodiment, said compound of formula (I) is used for killing cancer stem cells and/or cancer progenitor cells.

It is also an object of the present invention a compound having the following formula (IIa) 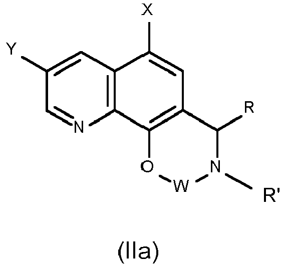 wherein
X is selected from H and halogen;
Y is selected from the group consisting of H, halogen, O-C₁-C₈alkyl, linear or branched, O-C₅-C₁₂aryl, O-C₁-C₈acyl, linear or branched, and O-C₃-C₈cycloalkyl;
W is selected from CH₂ and CO,
R is selected from H and C₆aryl,
R' is selected from the group consisting of: H; C₁-C₈alkyl, linear or branched; C₁-C₈acyl, linear or branched; C₃-C₈ cycloalkyl or heterocycloalkyl; C₅-C₁₄aryl or heteroaryl; CH(COOCH₃)(CH₂phenyl) and C₁-C₈alkylene-R²,
   wherein R² is selected from the group consisting of H, C₁-C₈alkyl, linear or branched, C₁-C₈acyl, linear or branched, C₃-C₈cycloalkyl or heterocycloalkyl, C₅-C₁₀aryl or heteroaryl,
   said heterocycloalkyl and heteroaryl containing one or more heteroatoms selected from N, S and O in a monocyclic or polycyclic system,
   said cycloalkyl, heterocycloalkyl, aryl or heteroaryl being optionally substituted on any position of the ring with one or more substituents selected from the group consisting of halogen, C₁-C₄alkyl, linear or branched, O-C₁-C₄alkyl, linear or branched, C₁-C₄acyl, linear or branched, an amino group optionally substituted with one or two C₁-C₄alkyl groups, SH and OH,
   wherein
when W is CH₂, R is H and X is halogen, R' is selected from the group consisting of C₁-C₈alkylene-indolyl and totally saturated cycloalkyl, optionally substituted,
the enantiomers, diastereoisomers, and mixtures thereof,
or a pharmaceutically acceptable salt, hydrate or solvate thereof.

Preferably, W is CH₂.

More preferably, W is CH₂, R is H and X is H. Even more preferably, in this embodiment, R' is selected from cycloalkyl and C₁-C₈alkylene-R² wherein R² is preferably phenyl.

It is also an object of the present invention a compound having the following formula (IIb) 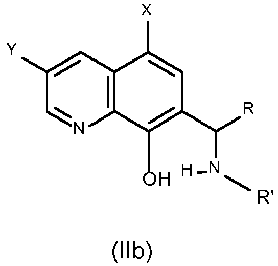 wherein
X is selected from H and halogen;
Y is selected from the group consisting of H, halogen, O-C₁-C₈alkyl, linear or branched, O-C₅-C₁₂aryl, O-C₁-C₈acyl, linear or branched, and O-C₃-C₈cycloalkyl;
R is selected from H and C₆aryl;
R' is selected from the group consisting of: H; C₁-C₈alkyl, linear or branched; C₃-C₈ cycloalkyl or heterocycloalkyl; C₅-C₁₄aryl or heteroaryl; CH(COOCH₃)(CH₂phenyl) and C₁-C₈alkylene-R²,
   wherein R₂ is selected from the group consisting of H, C₁-C₈alkyl, linear or branched, C₃-C₈cycloalkyl or heterocycloalkyl, C₅-C₁₀aryl or heteroaryl,
   said heterocycloalkyl and heteroaryl containing one or more heteroatoms selected from N, S and O in a monocyclic or polycyclic system
   said cycloalkyl, heterocycloalkyl, aryl or heteroaryl being optionally substituted on any position of the ring with one or more substituents selected from the group consisting of halogen, C₁-C₄alkyl, linear or branched, O-C₁-C₄alkyl, linear or branched, an amino group optionally substituted with one or two C₁-C₄alkyl groups, SH and OH,
   wherein
when X is H, R' is selected from the group consisting of totally saturated cycloalkyl, optionally substituted, and C₁-C₈alkylene-R²
the enantiomers, diastereoisomers, and mixtures thereof,
or a pharmaceutically acceptable salt, hydrate or solvate thereof.

When X is H, R' is preferably C₁-C₈alkylene-R² wherein R² is phenyl.

Preferably, X is H, R is a phenyl, more preferably a dichlorophenyl, and R' is C₁-C₈alkylene-R², R² being preferably a phenyl.

A process for the preparation of the compound of formula (IIa) or (IIb), as will be better defined below, is also an object of the present invention.

It is also an object of the invention the compound of formula (IIa) for use as a medicament.

A further object of the present invention is the compound of formula (IIa) for use in the treatment of a disease which can be improved or prevented by the inhibition of Gli1. For example, said disease can be a tumor.

A further object of the invention is a pharmaceutical composition containing as active ingredient a compound of formula (IIa) and at least one pharmaceutically acceptable vehicle and/or excipient.

It is also an object of the invention the compound of formula (IIb) for use as a medicament.

A further object of the present invention is a compound of formula (IIb) for use in the treatment of a disease which can be improved or prevented by the inhibition of Gli1. For example, said disease can be a tumor.

A further object of the invention is a pharmaceutical composition containing as active ingredient a compound of formula (IIb) and at least one pharmaceutically acceptable vehicle and/or excipient.

### Detailed description of the invention

### Definitions

Within the meaning of the present invention, the term "alkyl" refers to linear or branched alkyl groups having preferably from 1 to 8 carbon atoms, more preferably 1 to 3 carbon atoms. An alkyl group can be for example methyl, ethyl, *n*-propyl, *i*-propyl, *n*-butyl, *t*-butyl.

Within the meaning of the present invention, the term "cycloalkyl" refers to a saturated or partially unsaturated carbocyclic group of 3 to 8 carbon atoms. An aromatic group is not intended. The cycloalkyl group can comprise a single ring or multiple condensed rings. Examples of "C₃-C₈-cycloalkyl" include cyclopropyl, cyclobutyl, cyclopentyl, cyclohexyl, cycloheptyl, cyclooctyl. A partially unsaturated cycloalkyl is also intended, for example a cyclohexenyl.

Within the meaning of the present invention, the terms "heterocycloalkyl" and "heterocycle" are herein used as synonyms and refer to a saturated or partially unsaturated (but not aromatic) ring containing one or more nitrogen, sulphur or oxygen atoms. The heterocycloalkyl can comprise a single ring or multiple condensed rings. Preferred heterocycloalkyl include pyrrolidine, piperidine, piperazine, morpholine and tetrahydropyran.

Within the meaning of the present invention, the term "aryl" refers to aromatic rings, for example phenyl, naphthyl, thienyl and indolyl, also partially hydrogenated, for example tetrahydronaphthyl. The aryl group can comprise a single ring or multiple condensed rings.

Within the meaning of the present invention, the term "heteroaryl" refers to aromatic rings, also partially hydrogenated, containing one or more nitrogen, sulphur or oxygen atoms, for example indole. The heteroaryl group can comprise a single ring or multiple condensed rings.

Within the meaning of the present invention, the term "acyl" refers to groups derived by the removal of one or more hydroxyl groups from a carboxylic acid. It contains a double bonded oxygen atom and an alkyl group, linear or branched.

Within the meaning of the present invention, the term "alkylene" refers to a divalent alkyl group having two points of attachment within the compound of the invention.

Within the meaning of the present invention, the term "substituted" means an organic group as defined above (e.g., an alkyl group) in which one or more bonds to a hydrogen atom contained therein are replaced by a bond to non-hydrogen atoms. Substituted groups also include groups in which one or more bonds to a carbon(s) or hydrogen(s) atom are replaced by one or more bonds, including double or triple bonds, to a heteroatom. Thus, a substituted group is substituted with one or more substituents, unless otherwise specified.

Within the meaning of the present invention, cancer stem cells (CSCs) are cancer cells that possess characteristics associated with normal stem cells, in particular the ability to differentiate into all cell types found in a particular cancer sample and to self-renew indefinitely.

Within the meaning of the present invention, cancer progenitor cells are early descendants of cancer stem cells that can differentiate to form one or more kinds of cells, but cannot divide and self-renew indefinitely.

### Figures

Figure 1. Expression of Gli1 protein in NIH3T3 cells treated with Gli inhibitors. Western blotting of Gli1 protein in NIH3T3 cells treated with GANT61 (5 µM) and with each compound (1 µM) for 48 h.
Figure 2. Effects of SST0892AA1 on cancer cell growth. Example of dose-response curves in A375 and DAOY cells treated with increasing doses of SST0892AA1 for 72 h.

### Use of compounds of formula (I)

It is an object of the invention the use of compound of formula (I), as above described, for the treatment of diseases which can be improved or prevented by the inhibition of the Gli1 protein.

Indeed, it has been found that such compounds are able to inhibit expression of Gli1 and thus can be successfully used, in particular, in the treatment of tumors wherein Gli1 and/or the HH pathway is involved.

In the compound of formula (I), when W is CH₂ or CO, the dashed lines indicate single bonds, Z is O and A is N.

In the compound of formula (I), when W is absent, the dashed lines indicate absent bonds, Z is OH and A is NH.

In the compounds of formula (I) Y is preferably selected from H and a halogen, preferably fluorine.

According to the present invention, halogen is selected from the group consisting of: fluorine (F), chlorine (CI), bromine (Br) and iodine (I). Preferably, it is chlorine or fluorine.

When an aryl, heteroaryl, cycloalkyl or heterocycloalkyl is present, it can be linked to the rest of the molecule on any position of the ring(s).

In some embodiments, in the compound of formula (I), R is an aryl, preferably a phenyl, optionally substituted.

In some preferred embodiments, in the compound of formula (I), R' is an acyl group, for example a COC₁-C₄alkyl group.

In other preferred embodiments, in the compound of formula (I), R' is a C₁-C₈alkylene-R² group, preferably a C₁-C₄alkylene-R² group, wherein R² is preferably chosen from a cycloalkyl, preferably a cyclohexyl, and an aryl, preferably a phenyl or an indole.

For amino group optionally substituted with one or two C₁-C₄alkyl group, it is intended a NH₂ group wherein any of the hydrogen can be substituted with a C₁-C₄alkyl group, linear or branched, for example one or two methyl or one or two ethyl groups. For example, said amino group can be selected from NH₂, N(CH₃)₂ and N(CH₂CH₃)₂.

Any nitrogen group can be protected. The protective group is preferably selected from tert-butoxycarbonyl, benzyloxycarbonyl and 9-fluorenylmethyloxycarbonyl.

In an embodiment of the invention, compounds of formula (I) for the use according to the present invention are those in which the dashed bonds represent single bonds and W is present and chosen from CH₂ and CO, and a third cycle is formed. In this embodiment, A is N and Z is O.

In this embodiment, a first group of compounds are those in which W is CH₂, X is halogen, preferably chlorine, Y is H and R is H, R' having any of the meanings above disclosed. Preferably, R' is cycloalkyl, or C₁-C₄-alkyl or C₁-C₄-alkylene-R² wherein R² can have any of the meanings above defined, said R² preferably being a cycloalkyl, optionally partially unsaturated, an heterocycloalkyl or an aryl, said aryl being preferably a phenyl or an indolyl. When R' is cycloalkyl it is preferably cycloheptyl or cyclohexyl.

A second group of compounds are those in which W is CH₂ or CO, X is H, R is H and Y and R' can have any of the meanings above disclosed. Preferably, Y is H or halogen, preferably fluorine. Preferably, R' is a cycloalkyl, an aryl, CH(COOCH₃)(CH₂phenyl), or C₁-C₄-alkylene-R² wherein R² can have any of the meanings above defined, preferably being a cycloalkyl, an heterocycloalkyl or an aryl, said aryl being preferably a phenyl, optionally substituted, for example with O-alkyl, or an indole. When R' is a cycloalkyl it is preferably a cyclohexyl or a cycloheptyl.

A third group of compounds are those in which W is CH₂ or CO, X is H, R is aryl, optionally substituted, preferably with one or more halogens, and Y and R' can have any of the meanings above disclosed. Preferably, R is 2,4-dichlorophenyl. Preferably, R' is C₁-C₄-alkylene-R² wherein R² can have any of the meanings above defined, preferably being a phenyl.

In another embodiment of the invention, compounds of formula (I) for the use according to the present invention are those in which W is absent. In this embodiment, A is NH and Z is OH.

In this embodiment, a first group of compounds are those in which X is halogen, preferably chlorine, R is aryl, and Y and R' have any of the meanings above disclosed. Y is preferably H. R is preferably a phenyl, optionally substituted on any position; in some preferred embodiments, said phenyl is substituted by one or two halogens, preferably chlorine, by one or two O-alkyl groups, preferably O-methyl groups, by one or two alkyl groups or by an N(CH₂-CH₃)₂ group. R' is preferably an acyl group, more preferably a CO-C₁-C₄-alkyl group.

A second group of compounds are those in which X is halogen, preferably chlorine, R is H and Y and R' can have any of the meanings above disclosed. Y is preferably H. R' is preferably C₁-C₄-alkylene-R² wherein R² can have any of the meanings above defined, preferably being a phenyl.

A third group of compounds are those in which X is H, R is aryl and Y and R' can have any of the meanings above disclosed. Y is preferably H. R is preferably a phenyl, optionally substituted on any position; in some preferred embodiments, said phenyl is substituted by one or two halogens, preferably chlorine, or by one or two O-alkyl groups, preferably O-methyl groups. R' is preferably selected from an acyl group, more preferably a CO-C₁-C₄-alkyl group, and C₁-C₄-alkylene-R² wherein R² can have any of the meanings above defined, preferably being a phenyl.

When one or more chiral centers are present in the compounds of the present invention, the individual isomers, enantiomers and diastereoisomers, and mixtures thereof (e.g., racemates) are intended to be encompassed by the present invention.

The pharmaceutical acceptable salts of the compound of formula (I) for the use of the invention are also included in the scope of the invention.

Also, pharmaceutically acceptable hydrates or solvates of the compound of formula (I) for the use of the invention are included in the scope of the present invention. In particular, they can be any hydrate or solvate commonly used in the art.

### Compounds of formula (IIa and (IIb)

Compounds of formula (IIa), as above reported, are also objects of the present invention.

Such compounds are able to inhibit expression of Gli1 and thus can be successfully used, in particular, in the treatment of tumors wherein Gli1 and/or the HH pathway is involved.

In the compounds of formula (IIa) Y is preferably selected from H and a halogen, preferably fluorine.

According to the present invention, halogen is selected from the group consisting of: fluorine (F), chlorine (CI), bromine (Br) and iodine (I). Preferably, it is chlorine or fluorine.

In compounds of formula (IIa), R is selected from H and a phenyl, optionally substituted, preferably with one or two halogens. Preferably, R is a dichlorophenyl.

In preferred embodiments, in a compound of formula (IIa), R' is a C₁-C₈alkylene-R² group, preferably a C₁-C₄alkylene-R² group, wherein R² is preferably chosen from a cycloalkyl, preferably a cyclohexyl, and an aryl, preferably a phenyl or an indole.

In other embodiments, R' is a cycloalkyl, for example a cyclohexyl or a cycloheptyl.

Any nitrogen group can be protected. The protective group is preferably selected from tert-butoxycarbonyl, benzyloxycarbonyl and 9-fluorenylmethyloxycarbonyl.

A first group of compounds of formula (IIa) are those in which W is CH₂, X is halogen, preferably chlorine, and R' is selected from the group consisting of C₁-C₄alkylene-indolyl, cycloheptyl and cyclohexyl, optionally substituted in any position of the ring, preferably with a methyl. Y can have any of the meanings above defined, preferably being H. In a preferred embodiment, R' is (CH₂)₂-indolyl. The indolyl, when present, can be linked to the alkyl chain on any position of the ring.

A second group of compounds are those in which W is CO, X is H, and Y and R' can have any of the meanings above defined. Preferably, Y is H or halogen, preferably fluorine. Preferably, R' is C₁-C₄-alkylene-R², preferably a C₂-alkylene-R², wherein R² can have any of the meanings above defined, preferably being an aryl, said aryl being preferably a phenyl, optionally substituted.

A third group of compounds are those in which W is CH₂, X is H, Y can have any of the meanings above defined, preferably being H or halogen, preferably fluorine, and R' is selected from the group consisting of cycloalkyl, tetrahydronaphthalene, CH(COOCH₃)(CH₂phenyl), and C₁-C₈-alkylene-R² wherein R² can have any of the meanings above defined, preferably being H, a cycloalkyl, an heterocycloalkyl or an aryl, said aryl being preferably an indolyl or a phenyl, optionally substituted, for example with a O-methyl. Said cycloalkyl is preferably a cyclohexyl or a cycloheptyl.

A fourth group of compounds are those in which W is CH₂ or CO, X is H, R is aryl, optionally substituted, preferably with one or two halogens, and Y and R' can have any of the meanings above disclosed. Preferably, R is 2,4-dichlorophenyl. Preferably, R' is C₁-C₄-alkylene-R² wherein R² can have any of the meanings above defined, preferably being a phenyl.

Compounds of formula (IIb), as above reported, are also objects of the present invention.

In the compounds of formula (IIb), R is selected from H and a phenyl, optionally substituted, preferably with one or two halogens. Preferably, R is a dichlorophenyl.

In preferred embodiments, in a compound of formula (IIb), R' is a C₁-C₈alkylene-R² group, preferably a C₁-C₄alkylene-R² group, wherein R² is preferably chosen from a cycloalkyl, preferably a cyclohexyl, and an aryl, preferably a phenyl or an indole.

A first group of compounds of formula (IIb) are those in which X is halogen, preferably chlorine, R is H, and R' is C₁-C₈-alkylene-phenyl, preferably a C₂-alkylene-phenyl.

A second group of compounds of formula (IIb) are those in which X is H, R is phenyl, preferably 2,4-dichlorophenyl, and R' is C₁-C₈-alkylene-phenyl, preferably a C₂-alkylene-phenyl.

When one or more chiral centers are present in the compounds of the present invention, the individual isomers, enantiomers and diastereoisomers, and mixtures thereof (e.g., racemates) are intended to be encompassed by the present invention.

The pharmaceutical acceptable salts of the compound of formula (IIa) and (IIb) are included in the scope of the invention.

Pharmaceutical acceptable salts are salts which retain the biological activity of the compound and are derived from known pharmacologically acceptable basis. Examples of pharmaceutically acceptable salts are, for example, those from compounds of formula (I), (IIa) or (IIb) and inorganic bases, such as sodium, potassium, calcium and aluminum hydroxides, as well as those with organic bases such as, for example, lysine, arginine, N-methyl-glucamine, triethylamine, triethanolamine, dibenzylamine, methylbenzylamine, d-(2-ethyl-hexyl)-amine, piperidine, N-ethylpiperidine, N,N-di-ethylaminoethylamine, N-ethylmorpholine, 3-phenethylamine, N-benzyl-3-phenethylamine, N-benzyl-N,N-dimethylamine. A preferred salt is sodium salt.

Also, pharmaceutically acceptable hydrates or solvates of the compound of formula (IIa) and (IIb) are included in the scope of the present invention. In particular, they can be any hydrate or solvate commonly used in the art.

Pharmaceutically acceptable salts and their preparation are within the common knowledge of the skilled person and general reference is made to the relevant literature, such as, for example Pharmaceutical Salts and Co-crystals, Johan Wouters, Luc Quéré, Royal Society of Chemistry, 2011.

Exemplary compounds of formula (I) for the use according to the present invention are the followings:

**Table 1. Compounds for the use according to the present invention.**

| Internal Code | Compound name | Compound structural formula | CAS Number (for known compounds) |
|---|---|---|---|
| SST0687 | N-[(5-Chloro-8-hydroxy-quinolin-7-yl)-(3,4-dimethoxy-phenyl)-methyl]-acetamide | 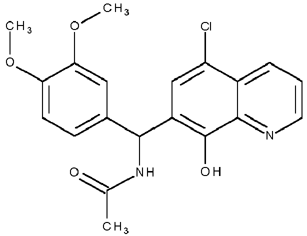 | 332939-52-3 |
| SST0689 | N-[(5-Chloro-8-hydroxy-quinolin-7-yl)-(2,4-dichlorophenyl)-methyl]-butyramide | 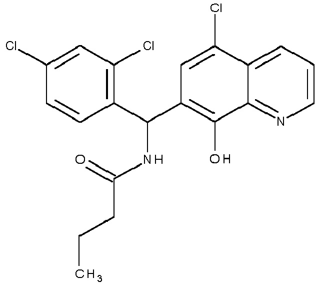 | 333318-59-5 |
| SST0769 | Propanamide, N-[(5-chloro-8-hydroxy-7-quinolinyl)(3,4-dimethoxyphenyl)methyl]-2-methyl- | 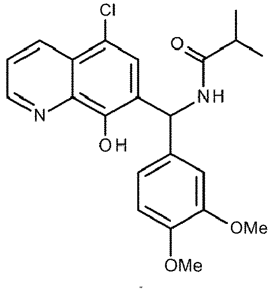 | 333318-54-0 |
| SST0770 | Butanamide, N-[(5-chloro-8-hydroxy-7-quinolinyl)(4-chlorophenyl)methyl]- | 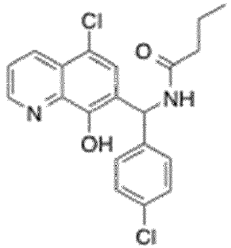 | 333318-55-1 |
| SST0771 | Butanamide, N-[(5-chloro-8-hydroxy-7-quinolinyl)[4-(diethylamino)phenyl] methyl]- | 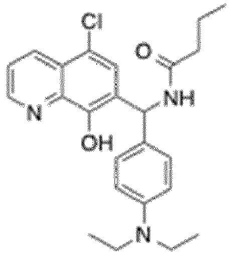 | 333318-69-7 |
| SST0772 | Butanamide, N-[(5-chloro-8-hydroxy-7-quinolinyl) phenylmethyl]- | 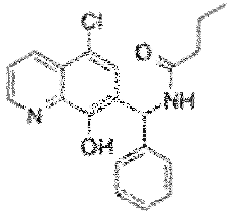 | 333315-23-4 |
| SST0773 | Butanamide, N-[(5-chloro-8-hydroxy-7-quinolinyl)(4-methylphenyl)methyl]- | 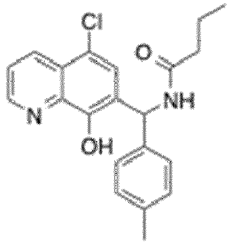 | 333318-49-3 |
| SST0774 | Pentanamide, N-[(5-chloro-8-hydroxy-7-quinolinyl)[4-(1-methylethyl)phenyl]methyl]- | 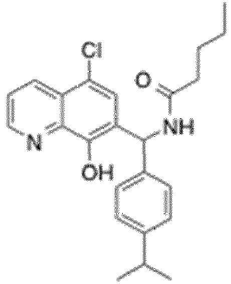 | 333318-67-5 |
| SST0775 | Butanamide, N-[(5-chloro-8-hydroxy-7-quinolinyl)(2-chlorophenyl)methyl]- | 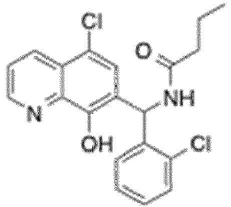 | 333318-57-3 |
| SST0776 | Butanamide, N-[(2,4-dichlorophenyl)(8-hydroxy-7-quinolinyl)methyl]- | 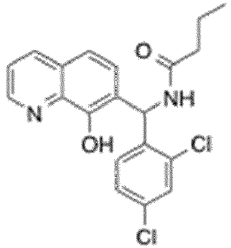 | 332939-26-1 |
| SST0777 | Pentanamide, N-[(3,4-dimethoxyphenyl)(8-hydroxy-7-quinolinyl)methyl] | 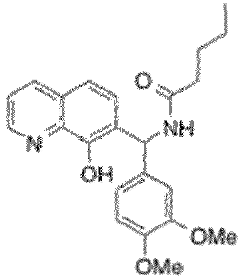 | 332173-76-9 |
| SST0791 | 2H-Pyrido[3,2-h]-1,3-benzoxazine, 6-chloro-3-[2-(1-cyclohexen-1-yl)ethyl]-3, 4-dihydro- | 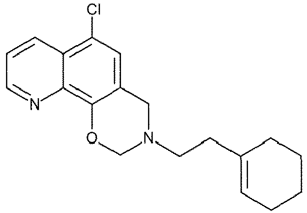 | 438486-44-3 |
| SST0792 | 2H-Pyrido[3,2-h]-1,3-benzoxazine, 6-chloro-3,4-dihydro-3-[3-(4-morpholinyl) propyl]- | 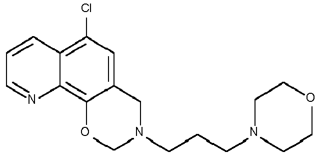 | 438486-41-0 |
| SST0793 | 2H-Pyrido[3,2-h]-1,3-benzoxazine, 3-butyl-6-chloro-3,4-dihydro- | 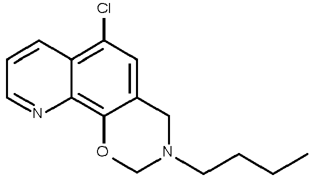 | 537010-87-0 |
| SST0794 | 2H-Pyrido[3,2-h]-1,3-benzoxazine, 6-chloro-3,4-dihydro-3-(2-phenylethyl)- | 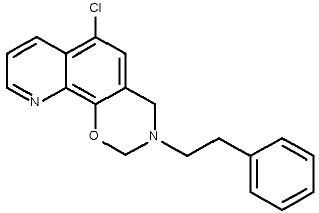 | 501017-82-9 |
| SST0823 | 3-phenethyl-3,4-dihydro-2H-[1,3]oxazino[5,6-h]quinoline | 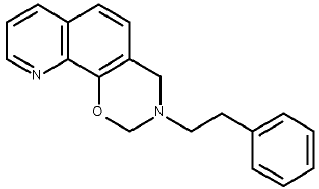 | |
| SST0824 | 4-(2,4-dichlorophenyl)-3-phenethyl-3,4-dihydro-2H-[1,3]oxazino[5,6-h]quinoline | 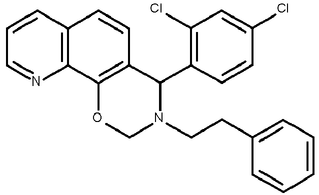 | |
| SST0825 | 3-phenethyl-3,4-dihydro-2H-[1,3]oxazino[5,6-h]quinolin-2-one | 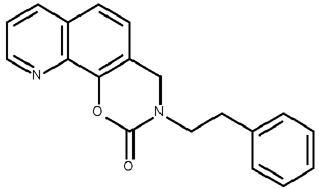 | |
| SST0826 | 4-(2,4-dichlorophenyl)-3-phenethyl-3,4-dihydro-2H-[1,3]oxazino[5,6-h]quinolin-2-one | 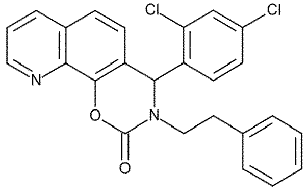 | |
| SST0827 | 7-((2,4-dichlorophenyl)(phenethyla mino)methyl)quinolin-8-ol | 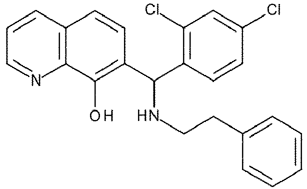 | |
| SST0888 | 3-(2-(1H-indol-3-yl)ethyl)-6-chloro-3,4-dihydro-2H-[1,3]oxazino[5,6-h]quinoline | 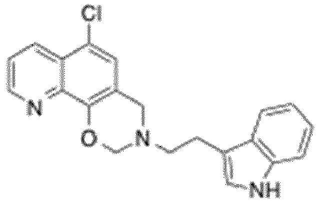 | |
| SST0889 | 5-chloro-7-[(phenethylamino)methyl]qu inolin-8-ol | 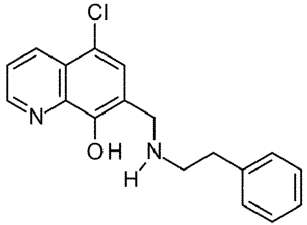 | |
| SST0890 | 3-(2-(1H-indol-3-yl)ethyl)-3,4-dihydro-2H-[1,3]oxazino[5,6-h]quinoline | 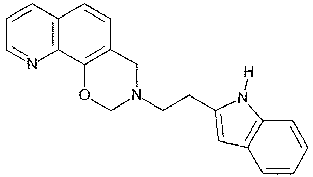 | |
| SST0891 | 8-fluoro-3-phenethyl-3,4-dihydro-2H-[1,3]oxazino[5,6-h]quinoline | 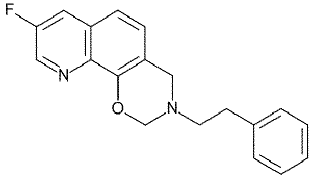 | |
| SST0892 | 3-cyclohexyl-3,4-dihydro-2H-[1,3]oxazino[5,6-h]quinoline | 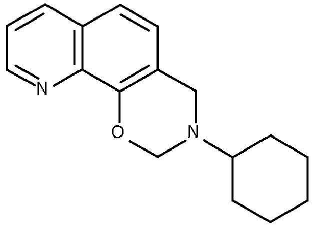 | |
| SST0893 | 3-(1,2,3,4-tetrahydronaphthalen-2-yl)-3,4-dihydro-2H-[1,3]oxazino[5,6-h]quinoline | 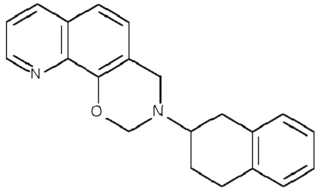 | |
| SST0910 | 3-(4-methoxybenzyl)-3,4-dihydro-2H-[1,3]oxazino[5,6-h]quinoline | 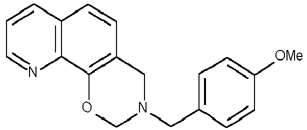 | |
| SST0911 | Methyl (S)-2-(2H-[1,3]oxazino[5,6-h]quinolin-3(4H)-yl)-3-phenylpropanoate | 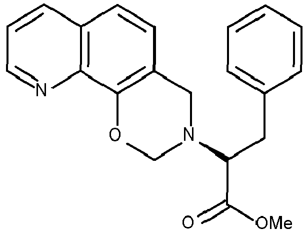 | |
| SST0912 | 3-(cyclohexylmethyl)-3,4-dihydro-2H-[1,3]oxazino[5,6-h]quinoline | 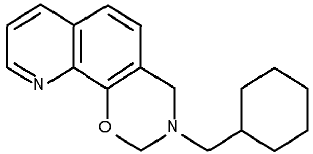 | |
| SST0913 | 3-cycloheptyl-3,4-dihydro-2H-[1,3]oxazino[5,6-h]quinoline | 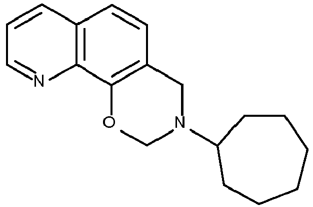 | |
| SST0925 | 3-cyclohexyl-8-fluoro-3,4-dihydro-2H-[1,3]oxazino[5,6-h]quinoline | 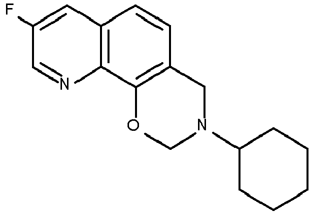 | |
| SST0926 | 6-ch loro-3-cycloheptyl-3,4-dihydro-2H-[1,3]oxazino[5,6-h]quinoline | 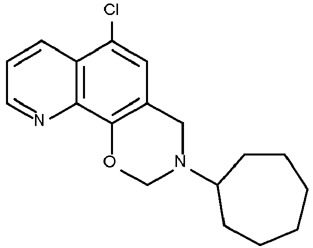 | |
| SST0927 | 6-chloro-3-(4-methylcyclohexyl)-3,4-dihydro-2H-[1,3]oxazino[5,6-h]quinoline | 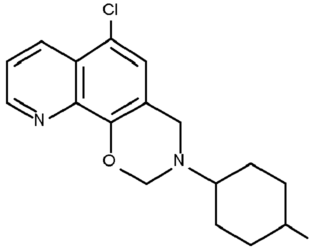 | |

Preferred compounds of formula (I) for the use according to the present invention are the followings:
Pentanamide, N-[(5-chloro-8-hydroxy-7-quinolinyl)[4-(1-methylethyl)phenyl]methyl]-(SST0774),
Butanamide, N-[(2,4-dichlorophenyl)(8-hydroxy-7-quinolinyl)methyl]-(SST0776),
Pentanamide, N-[(3,4-dimethoxyphenyl)(8-hydroxy-7-quinolinyl)methyl]-(SST0777),
N-[(5-Chloro-8-hydroxy-quinolin-7-yl)-(3,4-dimethoxy-phenyl)-methyl]-acetamide (SST0687),
N-[(5-Chloro-8-hydroxy-quinolin-7-yl)-(2,4-dichloro-phenyl)-methyl]-butyramide (SST0689),
Butanamide, N-[(5-chloro-8-hydroxy-7-quinolinyl)[4-(diethylamino)phenyl]methyl]-(SST0771),
3-phenethyl-3,4-dihydro-2*H*-[1,3]oxazino[5,6-*h*]quinoline (SST0823),
3-cyclohexyl-3,4-dihydro-2*H*-[1,3]oxazino[5,6-*h*]quinoline (SST0892), and
7-((2,4-dichlorophenyl)(phenethylamino)methyl)quinolin-8-ol (SST0827).

The following compounds of formula (IIa), already reported in Table 1 above, are also objects of the present invention:
3-phenethyl-3,4-dihydro-2*H*-[1,3]oxazino[5,6-*h*]quinoline (SST0823),
4-(2,4-dichlorophenyl)-3-phenethyl-3,4-dihydro-2*H*-[1,3]oxazino[5,6-*h*]quinoline (SST0824),
3-phenethyl-3,4-dihydro-2*H*-[1,3]oxazino[5,6-*h*]quinolin-2-one (SST0825),
4-(2,4-dichlorophenyl)-3-phenethyl-3,4-dihydro-2*H-*[1,3]oxazino[5,6-*h*]quinolin-2-one (SST0826),
3-(2-(1*H*-indol-3-yl)ethyl)-6-chloro-3,4-dihydro-2*H*-[1,3]oxazino[5,6-*h*]quinoline (SST0888),
3-(2-(1*H*-indol-3-yl)ethyl)-3,4-dihydro-2*H*-[1,3]oxazino[5,6-*h*]quinoline (SST0890),
8-fluoro-3-phenethyl-3,4-dihydro-2*H*-[1,3]oxazino[5,6-*h*]quinoline (SST0891),
3-cyclohexyl-3,4-dihydro-2*H*-[1,3]oxazino[5,6-*h*]quinoline (SST0892),
3-(1,2,3,4-tetrahydronaphthalen-2-yl)-3,4-dihydro-2*H*-[1,3]oxazino[5,6-*h*]quinoline (SST0893),
3-(4-methoxybenzyl)-3,4-dihydro-2*H*-[1,3]oxazino[5,6-*h*]quinoline (SST0910),
Methyl (S)-2-(2*H*-[1,3]oxazino[5,6-*h*]quinolin-3(4*H*)-yl)-3-phenylpropanoate (SST0911),
3-(cyclohexylmethyl)-3,4-dihydro-2H-[1,3]oxazino[5,6-h]quinoline (SST0912),
3-cycloheptyl-3,4-dihydro-2*H*-[1,3]oxazino[5,6-*h*]quinoline (SST0913),
3-cyclohexyl-8-fluoro-3,4-dihydro-2*H*-[1,3]oxazino[5,6-*h*]quinoline (SST0925),
6-chloro-3-cycloheptyl-3,4-dihydro-2*H*-[1,3]oxazino[5,6-*h*]quinoline (SST0926),
6-chloro-3-(4-methylcyclohexyl)-3,4-dihydro-2*H*-[1,3]oxazino[5,6-*h*]quinoline (SST0927).

Among the above mentioned compounds of formula (IIa), preferred compounds are the followings:
3-phenethyl-3,4-dihydro-2*H*-[1,3]oxazino[5,6-*h*]quinoline (SST0823),
3-cyclohexyl-3,4-dihydro-2*H*-[1,3]oxazino[5,6-*h*]quinoline (SST0892),
3-(1,2,3,4-tetrahydronaphthalen-2-yl)-3,4-dihydro-2*H*-[1,3]oxazino[5,6-*h*]quinoline (SST0893),
3-(4-methoxybenzyl)-3,4-dihydro-2*H*-[1,3]oxazino[5,6-*h*]quinoline (SST0910),
Methyl (S)-2-(2*H*-[1,3]oxazino[5,6-*h*]quinolin-3(4*H*)-yl)-3-phenylpropanoate (SST0911) and
3-(cyclohexylmethyl)-3,4-dihydro-2*H*-[1,3]oxazino[5,6-*h*]quinoline (SST0912).

The following compounds of formula (IIb), already reported in Table 1 above, are also objects of the present invention:
7-((2,4-dichlorophenyl)(phenethylamino)methyl)quinolin-8-ol (SST0827),
5-chloro-7-[(phenethylamino)methyl]quinolin-8-ol (SST0889).

Among these, a preferred compound is
7-((2,4-dichlorophenyl)(phenethylamino)methyl)quinolin-8-ol (SST0827).

### Synthesis processes

The following compounds are commercially available:
(SST0687) N-[(5-Chloro-8-hydroxy-quinolin-7-yl)-(3,4-dimethoxy-phenyl)-methyl]-acetamide
(SST0689) N-[(5-Chloro-8-hydroxy-quinolin-7-yl)-(2,4-dichloro-phenyl)-methyl]-butyramide
(SST0769) Propanamide, N-[(5-chloro-8-hydroxy-7-quinolinyl)(3,4-dimethoxyphenyl)methyl]-2-methyl-
(SST0770) Butanamide, N-[(5-chloro-8-hydroxy-7-quinolinyl)(4-chlorophenyl)methyl]-
(SST0771) Butanamide, N-[(5-chloro-8-hydroxy-7-quinolinyl)[4-(diethylamino)phenyl]methyl]-
(SST0772) Butanamide, N-[(5-chloro-8-hydroxy-7-quinolinyl)phenylmethyl]-
(SST0773) Butanamide, N-[(5-chloro-8-hydroxy-7-quinolinyl)(4-methylphenyl)methyl]-
(SST0774) Pentanamide, N-[(5-chloro-8-hydroxy-7-quinolinyl)[4-(1-methylethyl)phenyl]methyl]-
(SST0775) Butanamide, N-[(5-chloro-8-hydroxy-7-quinolinyl)(2-chlorophenyl)methyl]-
(SST0776) Butanamide, N-[(2,4-dichlorophenyl)(8-hydroxy-7-quinolinyl)methyl]-
(SST0777) Pentanamide, N-[(3,4-dimethoxyphenyl)(8-hydroxy-7-quinolinyl)methyl]-
(SST0791) 2*H*-Pyrido[3,2-*h*]-1,3-benzoxazine, 6-chloro-3-[2-(1-cyclohexen-1-yl)ethyl]-3,4-dihydro-
(SST0792) 2*H*-Pyrido[3,2-*h*]-1,3-benzoxazine, 6-chloro-3,4-dihydro-3-[3-(4-morpholinyl)propyl]-
(SST0793) 2*H*-Pyrido[3,2-*h*]-1,3-benzoxazine, 3-butyl-6-chloro-3,4-dihydro-
(SST0794) 2*H*-Pyrido[3,2-*h*]-1,3-benzoxazine, 6-chloro-3,4-dihydro-3-(2-phenylethyl)-

These compounds are identified in Table 1 above by their CAS number and can be commercially obtained.

They can be synthesized by many methods known in the art for their synthesis and available to those skilled in the art of organic chemistry. In particular, for the compounds listed in the above Table 1 reference can be made to the respective CAS number wherein exemplary synthesis methods can be found. Different methods or variations of the known methods to prepare the compounds will be evident to those skilled in the art. Also, the skilled in the art can easily slightly alter the reagents and reaction conditions indicated by the state of the art to include any combination of substituents defined in formula (I) and not explicitly represented in the above exemplary compounds according to the general knowledge in the field. Reference can be made, for example, to the handbook "March's Advanced Organic Chemistry, Reactions, Mechanism and structures.", Michael B. Smith, Jerry March, last edition.

Compounds of formula (IIa) and (IIb) can be synthesized by many methods available to those skilled in the art of organic chemistry. General and exemplary synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Additionally, the various steps in the synthesis may be performed in an alternate sequence in order to give the desired compound or compounds.

Examples of compounds of formula (IIa) and (IIb) prepared by methods described in the general schemes are given in the intermediates and examples section set out hereinafter.

The compounds of formula (IIa) and (IIb) can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionality present on the molecule should be consistent with the transformations proposed.

It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene et al. (Protective Groups in Organic Synthesis, 4th Edition, Wiley- Interscience (2006).

Also, the skilled in the art can easily alter the reagents and reaction conditions exemplified in the schemes below to include any combination of substituents as defined above. Also, the skilled artisan can easily use interchangeable steps for each synthetic process and incorporate isolation and/or purification steps as deemed necessary.

All the conditions and reagents not explicitly mentioned below can be easily chosen by the skilled in the art according to the general knowledge in the field.

Starting materials useful for the preparing the compounds of formula (IIa) and (IIb) and intermediates therefor, are commercially available or can be prepared by well known synthetic methods.

The final products obtained by the synthesis described below may be purified using techniques commonly known to one skilled in the art such as preparatory chromatography, thin-layer chromatography, HPLC, or crystallization.

Exemplary processes for the synthesis of the compounds of formula (IIa) and (IIb) are herein described.

### Exemplary processes

Compounds of formula (IIa) and (IIb) can be obtained through the exemplary general process of scheme 2 below.

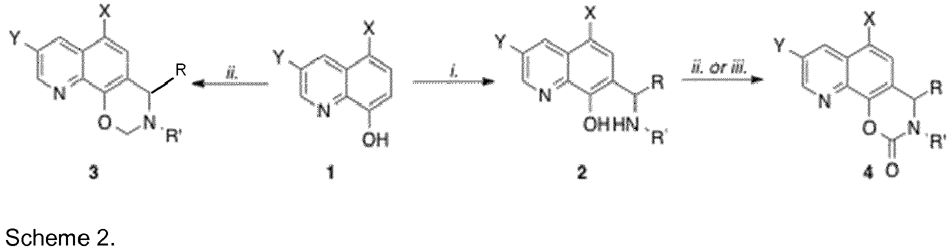

Compound 1 is a substituted 8-hydroxyquinoline wherein X and Y have the meanings above defined. Such compounds are commercially available or can be easily obtained by commercially available compounds through well known methods.

Compound of formula (IIb) (compound 2 in scheme 2 above), wherein W is absent, can be synthesized starting from the suitable compound 1 by treatment with the proper aldehyde (RCHO) and a suitable amine in a suitable solvent, preferably MeOH (step i in scheme 2). The reaction is typically carried out at room temperature for about 12 hours. Said suitable amine can be easily chosen by the skilled person depending on the final compound one wants to obtain, in particular depending on the meanings of R and R'. Such amines are commercially available or can be easily obtained by commercially available compounds through well known methods.

Tricyclic compounds of formula (IIa) (compound 3 in scheme 2 above) wherein W is CH₂, can be synthesized starting from the suitable compound 1 by stirring with the suitable amine in paraformaldehyde in a suitable solvent, preferably EtOH (step *ii* in scheme 2). The reaction is preferably carried out at reflux for about 6-12 hours. Said suitable amine can be easily chosen by the skilled person depending on the final compound one wants to obtain, in particular depending on the meanings of R and R'. Such amines are commercially available or can be easily obtained by commercially available compounds through well known methods.

Alternatively, compounds 3 can be obtained starting from the suitable compound 2, obtained as above described, by treatment with paraformaldehyde in a suitable solvent, preferably EtOH; the reaction is typically carried out at reflux overnight (step *ii* in scheme 2).

Compounds of formula (IIa) (compound 4 in scheme 2 above) wherein W is CO can be obtained from the suitable compound 2, obtained as above described, by treatment with carbonyldiimidazole (CDI) in a suitable solvent, for example tetrahydrofuran (THF) (step *iii* in scheme 2).

In an exemplary embodiment, compounds of formula (IIa) wherein W is CO can be obtained by adding CDI to the suitable compound 2, preferably in suitable solvents such as diisopropylamine (DIPEA) and THF. The mixture is typically refluxed overnight under N₂, quenched for example with HCl, for example 5% HCl, and extracted; extracts are washed with water and dried.

In another exemplary embodiment, compounds of formula (IIa) wherein W is CH₂, can be obtained by dissolving in a suitable solvent, such as EtOH, a suitable amine and a suitable aldehyde and refluxing for about 6 hours. 8-Hydroxyquinoline, optionally substituted, is then added to obtain the final desired compound.

In another exemplary embodiment, compounds of formula (IIb), can be obtained by mixing 8-hydroxyquinoline, optionally substituted, with a suitable amine in formaline and MeOH, or similar solvents. For example, for obtaining a compound wherein R' is C₂-alkylene-phenyl, phenylethylamine is added.

For obtaining a compound wherein R is aryl, a benzaldehyde, optionally substituted, can be used in the above mentioned processes.

For example, for obtaining a compound wherein R' is C₂-alkylene-phenyl, phenylethylamine is added. In another example, for obtaining compounds wherein R' is selected from the group consisting of cyclohexyl, cycloheptyl, tetrahydronaphthalene, CH(COOCH₃)(CH₂phenyl), and C₂alkylene-R² wherein R² can be phenyl, indole or cyclohexyl, the suitable amine can be chosen among the following: phenylethylamine, tryptamine, cyclohexylamine, 1,2,3,4-tetrahydronaphthalen-2-amine, 4-methoxybenzylamine, L-phenylalanine methylester, cyclohexylmethanamine, cycloheptylamine. In a further example, compounds wherein X is halogen, R is H and R' is selected from the group consisting of C₂alkylene-indolyl, cycloheptyl and cyclohexyl can be obtained by using one of the following amines: cycloheptylamine, 4-methyl-cyclohexylamine and adding 5-chloro-8-hydroxyquinoline.

For obtaining final compounds wherein Y is fluorine, 3-fluoro-8-hydroxyquinoline can be used.

All the transformations above described are only exemplary synthetic procedures already known in organic chemistry and well known to the experts in the field.

Any variation or combination of substituents of compounds of formula (IIa) and (IIb) which is within the scope of the present invention and is not explicitly shown or described in the above processes, can be easily obtained starting from the exemplary processes described above with suitable modifications, for example in the starting compounds or reagents, that are well within the knowledge of the skilled person in the field of organic chemistry. Reference can be made, for example, to the handbook "March's Advanced Organic Chemistry, Reactions, Mechanism and structures.", Michael B. Smith, Jerry March, last edition.

### Medical uses

It is an object of the invention the compound of formula (I) for use in the treatment of pathologies wherein Gli1 is involved.

In particular, the compounds of the invention can be used in the treatment of pathologies which can be prevented or ameliorated by the inhibition of Gli1. This means that such pathologies are caused or characterized by an increase in the activity of Gli1. More in particular, the compounds of the invention can be used for the treatment of tumors and metastasis. Indeed, activation of Gli1 leads to upregulation of many pro-proliferative, pro-survival and pro-angiogenic genes, leading to tumor growth and therapeutic resistance. Therefore, the inhibition of this protein in tumor and metastasis is particularly advantageous. In the review of Rimkus et al., 2016, already mentioned above, an overview of the human cancers wherein Gli1 is involved, and which can therefore benefit from the anti-Gli1 activity of the compounds of the invention, is reported.

In particular, compounds of formula (I) can be advantageously used in the treatment of tumors mediated by the HH signaling pathway.

Those skilled in the art would readily be able to determine whether a cancer is mediated by the HH signaling pathway or is caused or characterized by an increase in the activity of Gli1, for example by analyzing cancer cells using one of several techniques known in the art. Also, reference can be made to the cited works of Rimkus et al., 2016; Tang C.M. 2016 Oncotarget Oct 25; D'Amico D. 2016 Trends Mol. Med. 22: 851-862; Di Magno L. 2015 Biochim. Biophys Acta 1856: 62-72; Wang et al. Oncotarget 2016.

Exemplary tumors which can be treated by the compounds of the invention are: basal cell carcinoma (BCC), medulloblastoma (MB), glioma, pancreatic, colon, small cell lung cancer (SCLC), non-small cell lung cancer (NSCLC), prostate and ovarian cancers, melanoma and hematological malignancies, such as chronic myeloid leukemia (CML), acute lymphoblastic leukemia (ALL) and acute myeloid leukemia (AML). Preferred tumors are basal cell carcinoma and medulloblastoma.

It is also an object of the invention the use of compounds of formula (IIa) and (IIb) as a medicament, in particular for diseases which can benefit from an inhibition of the Gli1 protein. More in particular, they can be advantageously used in the treatment of tumors, for the reasons explained above. Indeed, it has been found that such compounds are able to inhibit expression of Gli1 and thus can be successfully used, in particular, in the treatment of tumors wherein Gli1 and/or the HH pathway is involved.

### Pharmaceutical compositions

Another object of the present invention is a pharmaceutical composition containing at least one compound of formula (IIa) or (IIb) as an active ingredient, typically in an amount such as to produce a significant therapeutic effect, together with pharmaceutically acceptable vehicles and/or excipients.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

Another object of the present invention is a pharmaceutical composition containing at least one compound of formula (I) as an active ingredient, typically in an amount such as to produce a significant therapeutic effect, together with pharmaceutically acceptable vehicles and/or excipients for use in the treatment of diseases which can be improved or prevented by the inhibition of the Gli1 protein.

The compositions according to the present invention contain, along with the active ingredient, at least one pharmaceutically acceptable vehicle or excipient. These may be particularly useful formulation coadjuvants, e.g. solubilising agents, dispersing agents, suspension agents, and emulsifying agents.

The pharmaceutical composition according to the invention comprising the compound of formula (I) or (IIa) or (IIb) can also contain one or more further active ingredients, for example chemotherapeutic agents. In particular, when the pharmaceutical composition is used for the treatment of a tumor, it preferably comprises also a chemotherapeutic agent. Indeed in some embodiments, the compounds of the invention can facilitate the tumor killing action of other agents and even have a synergic effect with chemotherapeutic agents. Therefore, the combination of the compounds of the invention with any chemotherapeutic agent can be particularly useful. The chemotherapeutic agent can be chosen among the known agents according to the general knowledge of the skilled person in the field. For example, said chemotherapeutic agent can be selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens. When said chemotherapeutic agent is a cytotoxic agent, it is preferably selected from the group consisting of melphalan, irinotecan, rituximab and lapatinib. When said chemotherapeutic agent is a proteasome inhibitor, it is preferably selected from the group consisting of bortezomib, carfilzomib and ixazomib. When said chemotherapeutic agent is an immunomodulatory agent, it is preferably selected from the group consisting of thalidomide, lenalidomide and pomalidomide.

According to the administration route chosen, the compositions will be in solid or liquid form, suitable for oral, parenteral or topical administration.

Generally, the compounds of this invention are administered in a "therapeutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. For any compound, the therapeutically effective dose can be estimated initially either in cell culture assays or in animal models, usually mice, rats, guinea pigs, rabbits, dogs, or pigs. The animal model may also be used to determine the appropriate concentration range and route of administration. Such information can then be used to determine useful doses and routes for administration in humans. In calculating the Human Equivalent Dose (HED) it is recommended to use the conversion table provided in Guidance for Industry and Reviewers document (2002, U.S. Food and Drug Administration, Rockville, Maryland, USA).

Generally, an effective dose will be from 0.01 mg/kg to 100 mg/kg, preferably 0.05 mg/kg to 50 mg/kg. For any compound, the therapeutically effective dose can be estimated initially either in cell culture assays or in animal models, usually mice, rats, guinea pigs, rabbits, dogs, or pigs. The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

Compositions may be administered individually to a patient or may be administered in combination with other agents, drugs or hormones. For example they can be used in combination with further active ingredients, such as for example anticancer drugs.

The medicament may also contain a pharmaceutically acceptable carrier, for administration of a therapeutic agent. Such carriers include antibodies and other polypeptides, genes and other therapeutic agents such as liposomes, provided that the carrier does not induce the production of antibodies harmful to the individual receiving the composition, and which may be administered without undue toxicity.

Suitable carriers may be large, slowly metabolized macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol.

Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intra-arterial, intramedullary, intrathecal, intraventricular, transdermal or transcutaneous applications, subcutaneous, intraperitoneal, intranasal, enteral, topical, sublingual, intravaginal or rectal means.

The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention is usually a minor component (from about 0.1 to about 50% by weight or preferably from about 1 to about 40% by weight) with the remainder being various vehicles or carriers and processing aids helpful for forming the desired dosing form. Dosage treatment may be a single dose schedule or a multiple dose schedule.

A further object of the invention is a process for the preparation of pharmaceutical compositions characterized by mixing one or more compounds of formula (IIa) or (IIb) with suitable excipients, stabilizers and/or pharmaceutically acceptable diluents.

It is also an object of the invention the use of such pharmaceutical compositions comprising at least a compound of formula (IIa) or (IIb) as a medicament, in particular in the treatment of a disease which can be improved or prevented by an activity of inhibition of Gli1. More in particular, cancer diseases can be treated by such pharmaceutical compositions.

Pharmaceutical compositions and preparations thereof are within the general knowledge and reference can be made to conventional manuals and national and European Pharmacopoeias.

The following examples further illustrate the invention.

### EXAMPLES

We describe a general procedure for the synthesis of three subclasses of compounds (**2, 3,** and **4** in Scheme 3 below).

Derivatives **2** have been synthesized starting from substituted 8-hydroxyquinolines by treatment with formalin and a proper amine in MeOH at room temperature for 12 hours obtaining **2** in good to excellent yields. The same starting material was used for the synthesis of the tricyclic compounds **3,** by stirring with the proper amine in paraformaldehyde and EtOH at reflux for 12 hours.

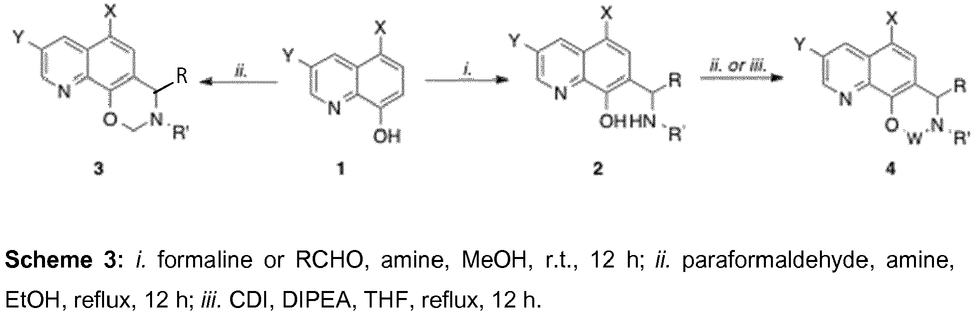

Derivatives **2** are used as the starting materials for the synthesis of **4** by treatment with paraformaldehyde in EtOH at reflux overnight (W = CH₂) or with CDI in THF (W = CO).

### Example 1

### General procedure for the synthesis of quinolin-8-ol derivatives 2 with R = H

The proper amine (1.1 mmol) and quinolin-8-ol derivative (1.1 mmol) were added to a solution of formaline (112 µL, 1.1 mmol) in MeOH (1 mL). The reaction mixture was stirred at room temperature for 12 h. The solid obtained was filtered in a Büchner funnel washing the solid with Et₂O (3 x 7 mL).

### 5-chloro-7-[(phenethylamino)methyl]quinolin-8-ol (SST0889AA1): amine = phenylethylamine

Yield: 30%. ¹HNMR (400 MHz, d₆DMSO, δ ppm): 8.80 (d, 2H; J = 3.6 Hz); 8.40 (d, 1H; J = 8.8 Hz); 7.44-7.16 (m, 6H), 5.35 (s, 1H); 4.01 (s, 2H); 2.97 (t, 2 H; J = 13.2 Hz); (t, 2H; J = 8.4 Hz).

ES-MS: 313 [M+H]⁺

### Example 2

### General procedure for the synthesis of quinolin-8-ol derivatives 2 with R = Ar

A mixture of 8-hydroxyquinoline derivative (1.1 eq.), a proper amine or amide (1 eq.) and aryl aldehyde component (3 eq.) were heated neat at 145-150°C for 3-6 h. Typically, the hydroxyquinoline was isolated by multiple trituration with hexanes and isopropyl ether followed by recrystallization from ethanol furnishing product in 14-80% yield.

7-((2,4-dichlorophenyl)(phenethylamino)methyl)quinolin-8-ol **(SST0827AA1):** amine = phenylethylamine, aldehyde = 2,4-dichlorobenzaldehyde ¹H NMR (400 MHz, CDCl₃, δ ppm) 8.85 (d, 1 H; *J* = 4.3 Hz), 8.05 (d, 1 H; *J* = 8.3 Hz), 7.44-7.02 (m, 6H), 5.56 (s, 1 H), 3.11-2.77 (m, 4H). ¹³C-NMR (100 MHz, CDCl₃, δ ppm) 152.10, 148.35, 138.87, 138.62, 137.12, 135.32, 130.15, 129.09, 128.26, 128.13, 127.91, 127.11, 126.91, 125.98, 121.27, 120.08, 117.33, 60.74, 49.18, 35.54.

### Example 3

### General procedure for the synthesis of derivatives 4 with R = H or 2,4-dichlorophenyl and W = CO

CDI (1.2 eq.) was added to a solution of **2** (1 eq.), and DIPEA (3 eq.) in THF. The reaction mixture was refluxed overnight under N₂. The reaction mixture was quenched with 5% HCl and extracted with CH₂Cl₂. The combined extracts were washed with water and dried over dry Na₂SO₄. After evaporation of the solvent under reduced pressure, the crude product was purified by recrystallization from acetone.

4-(2,4-dichlorophenyl)-3-phenethyl-3,4-dihydro-2*H-*[1,3]oxazino[5,6-*h*]quinolin-2-one **(SST0826AA1):** starting from 2 = SST0827¹H NMR (400 MHz, CDCl₃, δ ppm) 8.99 (d, 1H; *J* = 4.3 Hz), 8.08 (d, 1 H; *J =* 8.3 Hz), 7.50-7.02 (m, 10H), 5.95 (s, 1 H), 3.04-2.82 (m, 4H). ¹³C-NMR (100 MHz, CDCl₃, δ ppm) 150.67, 137.79, 136.67, 135.29, 134.91, 130.18, 129.32, 128.43, 128.33, 128.20, 126.19, 123.42, 122.71, 122.00, 118.25, 58.27, 49.18, 33.07.

3-phenethyl-3,4-dihydro-2*H*-[1,3]oxazino[5,6-*h*]quinolin-2-one **(SST0825AA1):** starting from 2 = SST0909 ¹H NMR (400 MHz, CDCl₃, δ ppm) 8.97 (d, 1 H; *J* = 4.2 Hz), 8.08 (d, 1 H; *J* = 8.2 Hz,), 7.49 (d, 1 H; *J* = 8.4 Hz), 7.42 (d, 1 H; *J* = 8.6 Hz), 7.32-7.13 (m, 4H), , 4.42 (s, 2H), 3.77 - 3.01 (m, 4H). ¹³C-NMR (100 MHz, CDCl₃, δ ppm) δ 150.44, 149.14, 138.05, 135.23, 128.41, 128.24, 128.01, 126.17, 122.87, 122.53, 121.56, 115.16, 50.95, 48.40, 32.77.

### Example 4

### General procedure for the synthesis of quinolin derivatives 3 with R = H

The proper amine (2.1 mmol) and paraformaldehyde (191 mg, 6.21 mmol) were dissolved in EtOH (10 mL) and refluxed for 6 h. The reaction mixture was cooled down to room temperature and the proper quinolin derivative (2.1 mmol). The reaction was stirred at room temperature for 12 h. The solvent was evaporated under vacuum and the crude mixture purified by flash chromatography (CH₂Cl₂/MeOH 9:1).

### 3-(2-(1H-indol-3-yl)ethyl)-6-chloro-3,4-dihydro-2H-[1,3]oxazino[5,6-h]quinoline (SST0888AA1): amine = tryptamine

Yield 16% ¹H⁻NMR: (400 MHz, , d₆DMSO, δ ppm): 10.60 (s, 1 H); 8.90 (s, 1 H); 8.44 (d, 1 H; J = 8.4 Hz); 8.26 (s, 1 H); 7.67 (d, 2H; J = 8.8 Hz); 7.55 (d, 2H; J = 8.4 Hz); 7.31 (d, 2H; J = 7.6 Hz); 7.20 (d, 2H; J = 7.6 Hz); 7.03 (d, 2H; J = 8 Hz); 6.97-6.87 (m, 6H); 3.95 (s, 2H); 3.66 (s, 2H); 3.86 (s, 2H); 2.69 (s, 3H). ES-MS: 364 [M+H]⁺

### 3-(2-(1H-indol-3-yl)ethyl)-3,4-dihydro-2H-[1,3]oxazino[5,6-h]quinoline (SST0890AA1): amine = tryptamine

Yield 10% ¹H-NMR: (400 MHz, CDCl₃, δ ppm): 10.59 (s, 1H); 8.79 (d, 1H; J = 3.6 Hz); 8.26 (d, 1 H; J = 8.4 Hz); 7.49 (m, 2H); 7.33 (q, 2H; J = 26.4 Hz); 7.19 (d, 1 H; J = 8 Hz); 6.91 (m, 2H); 3.95 (s, 2H); 3.63 (s, 2H); 2.85 (t, 2H; J = 10.8 Hz); 2.68 (d, 2H; J = 4.8 Hz). ES-MS: 330 [M+H]⁺

### 3-cyclohexyl-3,4-dihydro-2H-[1,3]oxazino[5,6-h]quinoline (SST0892AA1): amine = cyclohexylamine

Yield 35% ¹H-NMR: (400 MHz, CDCl₃, δ ppm): 8.84 (t, 1 H; J = 4.4 Hz); 8.02 (d, 1 H; J= 8 Hz); 7.27 (m, 2H); 7.10 (d, 1H; J = 8.4 Hz); 5.23 (s, 2H); 4.19 (s, 2H); 2.75 (q, 1H; J = 21.6 Hz); 1.99 (t, 2H J = 44.8 Hz); 1.68 (d, 2H; J = 12.4 Hz); 1.52 (d, 1 H J = 11.6 Hz); 1.25 (m, 6H) ES-MS: 269 [M+H]⁺

3-(1,2,3,4-tetrahydronaphthalen-2-yl)-3,4-dihydro-2*H*-[1,3]oxazino[5,6-*h*]quinoline **(SST0893AA1):** amine = 1,2,3,4-tetrahydronaphthalen-2-amine Yield 51% ¹H-NMR: (400 MHz, CDCl₃, δ ppm): 8.84 (d, 1 H; J = 3.2 Hz); 7.98 (d, 1 H; J = 8.4 Hz); 7.55 (t, 1 H; J = 8.8 Hz); 7.28 (q, 1 H; J = 12.4 Hz); 7.20 (d, 1 H; J = 8.4 Hz); 7.11 (q, 2H; J = 8.8 Hz); 7.02 (q, 2H; J = 4.4 Hz); 6.99 (d, 1H, J = 8.8 Hz); 5.25 (t, 2H; J = 14.4 Hz); 4.24 (d, 1H; J = 8 Hz); 4.15 (d, 1H; J = 17.6 Hz); 3.85 (d, 1H; J = 17.4 Hz); 2.67-2.60 (m, 2H); 2.04 (q, 1H; J = 14.8 Hz); 1.78 (t, 1H; J = 12 Hz); 1.60 (q, 2H; J = 12 Hz). ¹³C⁻NMR: (100 MHz, CDCl₃, δ ppm): 150.71; 149.35; 139.82; 138.40; 137.35; 135.88; 129.10; 128.40; 127.86; 126.85; 125.81; 125.27; 121.08; 120.81; 118.67; 83.43; 62.51; 46.01; 29.17; 28.10; 21.12. ES-MS: 317 [M+H]⁺

### 3-(4-methoxybenzyl)-3,4-dihydro-2H-[1,3]oxazino[5,6-h]quinoline (SST0910AA1): amine = 4-methoxybenzylamine

Eluent: PE/AcOEt (1:1); Yield 48%; ¹H-NMR: (400 MHz, CDCl₃, δ ppm): 8.86 (d, 1H; J = 4.4 Hz); 8.03 (d, 1 H; J = 8 Hz); 7.32 (q, 1 H; J = 12.4 Hz); 7.25 (q, 3H; J = 8,4 Hz) ; 7.04 (d, 1 H; J = 8.4 Hz); 6.82 (d, 2H; J = 8.4 Hz); 5.12 (s, 2H); 4.05 (d, 2H; J = 10 Hz); 3.89 (s, 2H); 3.75 (s, 3H). ¹³C⁻NMR: (100 MHz, CDCl₃, δ ppm): 158.97; 149.50; 149.37; 139.32; 135.92; 130.19; 130.09; 128.10; 126.17; 121.15; 119.00; 117.87; 113.80; 83.07; 55.21; 55.18; 49.42. ES-MS: 307 [M+H]⁺

### 3-phenethyl-3,4-dihydro-2H-[1,3]oxazino[5,6-h]quinoline (SST0823AA1): amine = phenylethylamine

Yield 30%; ¹H-NMR: (400 MHz, CDCl₃, δ ppm): 8.81 (t, 1 H; J = 4 Hz); 7.94 (d, 1 H; J = 8 Hz); 7.22-7.10 (m, 8H); 5.10 (s, 2H); 4.08 (s, 2H); 3.04 (t, 2H; J = 16 Hz); 2.85 (t, 2H; J = 16 Hz). ES-MS: 291[M+H]^{+ 13}C-NMR (100 MHz, CDCl₃, δ ppm) δ 148.98, 139.27, 138.93, 135.45, 128.24, 127.92, 125.70, 125.58, 120.74, 118.49, 117.66, 82.67, 52.96, 50.38, 34.48.

### 3-(cyclohexylmethyl)-3,4-dihydro-2H-[1,3]oxazino[5,6-h]quinoline (SST0912AA1): amine = cyclohexylmethylamine

Eluent PE/AcOEt (1:1); Yield 48% ¹H⁻NMR: (400 MHz, CDCl₃, δ ppm): 8.75 (t, 1H; J = 4.4 Hz); 7.90 (t, 1 H; J = 8.4 Hz); 7.23-7.15 (m, 2H); 6.99 (d, 1 H; J = 8 Hz); 5.00 (s, 2H); 3.98 (s, 2H); 2.52 (d, 2H; J = 7.2 Hz); 1.49-1.44 (m, 6H); 1.16-1.10 (m, 2H). ¹³C⁻NMR: (100 MHz, CDCl₃, δ ppm): 149.69; 149.33; 139.32; 135.87; 128.00; 126.16; 121.08; 118.66; 118.21; 84.04; 58.49; 51.04; 36.34; 31.38; 26.71; 26.01. ES-MS: 283 [M+H]⁺

### 3-cycloheptyl-3,4-dihydro-2H-[1,3]oxazino[5,6-h]quinoline (SST0913AA1): amine = cycloheptylamine

Eluent PE/AcOEt (1:1); Yield 87%; ¹H-NMR: (400 MHz, CDCl₃, δ ppm): 8.77 (d, 1H; J = 3.6 Hz); 7.92 (d, 1 H; J = 8.4 Hz); 7.22-7.11 (m, 2H); 5.11 (s, 2H); 4.08 (s, 2H); 2.89 (d, 1 H; J = 8.8 Hz); 1.40-1.35 (m, 6H). ES-MS: 283 [M+H]⁺ 305 [M+Na]⁺

### Methyl (S)-2-(2H-[1,3]oxazino[5,6-h]quinolin-3(4H)-yl)-3-phenylpropanoate (SST0911AA1): amine = L-phenylalanine methylester

Eluent PE/AcOEt (1:1); Yield 21%; ¹H-NMR: (400 MHz, CDCl₃, δ ppm): 8.84 (d, 2H; J = 4.3 Hz), 8.01 (d, 2H; *J* = 8.1 Hz), 7.30-7.25 (m, 2H), 7.25-7.05 (m, 4H), 5.24 (d, 1 H; *J* = 10.3Hz), 5.15 (d, 1H; *J* = 10.3 Hz), 4.41 (d, 1H; *J* = 17.0 Hz), 4.19 (d, 1 H; *J =* 17.1 Hz), 3.89 (dd, 1 H; *J* = 9.1, 6.1 Hz,), 3.32 (s, 3H), 3.23-3.00 (m, 2H). ¹³C NMR (100 MHz, CDCl₃, δ ppm) 172.65, 149.47, 137.03, 135.91, 129.20, 129.14, 128.39, 127.95, 126.88, 126.65, 125.45, 121.44, 121.23, 119.35, 82.01, 66.17, 51.66, 47.76, 36.80. ES-MS: 309 [M+H]⁺

### 8-fluoro-3-phenethyl-3,4-dihydro-2H-[1,3]oxazino[5,6-h]quinoline (SST0891AA1): amine = phenylethylamine

Eluent AcOEt, Yield 55%; ¹H NMR (400 MHz, CDCl₃, δ ppm) 8.67 (d, 1 H; *J* = 2.8 Hz), 7.54 (dd, 1 H; *J* = 9.0, 2.8 Hz,), 7.22-7.00 (m, 7H), 5.08 (s, 2H), 4.05 (s, 2H), 3.03-2.99 (m, 2H), 2.80-2.64 (m, 2H). ¹³C-NMR (100 MHz, CDCl₃, δ ppm) 157.87; 155.33; 149.89; 139.59; 136.34; 12848; 118.63; 117.36; 83.25; 53.36; 50.51; 34.85. ES-MS: 309 [M+H]⁺

### 3-cyclohexyl-8-fluoro-3,4-dihydro-2H-[1,3]oxazino[5,6-h]quinoline (SST0925AA1): amine = cyclohexylamine

Yield 50%; ¹H NMR (400 MHz, CDCl₃, δ ppm) 8.65 (dd, 1 H; *J* = 8.0, 2.8 Hz), 7.59 (dd, 1 H; *J* = 9.0, 2.8 Hz,), 7.22 (d, 1 H, 8.4 Hz); 7.12 (d, 1 H, 8.4 Hz), 5.23 (s, 2H), 4.18 (s, 2H), 2.78-2.71 (m, 1 H); 1.96 (d, 2H; J = 12 Hz); 1.69 (d, 2H; J = 12 Hz); 1.53 (d, 2H; J = 12 Hz) 1.22-1.06 (m, 4H). ES-MS: 287 [M+H]⁺

### 6-chloro-3-cycloheptyl-3,4-dihydro-2H-[1,3]oxazino[5,6-h]quinoline (SST0926AA1): amine = cycloheptylamine

Eluent PE/AcOEt (1:1); Yield 85%; ¹H-NMR: (400 MHz, CD₃OD, δ ppm): 8.70 (d, 1 H; J = 3.6 Hz); 8.31 (d, 1 H; J = 2.8 Hz); 7.39 (bs, 1 H); 7.17 (s, 1 H); 4.92 (s, 2H); 4.00 (s, 2H); 2.89 (bs, 1 H); 1.85-1.31 (m, 14H). ES-MS: 331 [M+H]⁺

### 6-chloro-3-(4-methylcyclohexyl)-3,4-dihydro-2H-[1,3]oxazino[5,6-h]quinoline (SST0927AA1): amine = 4-methylcyclohexylamine

Eluent PE/AcOEt (1:1); Yield 37%; ¹H-NMR: (400 MHz, CDCl₃, δ ppm): 8.74 (d, 1H; J = 3.6 Hz); 8.25 (d, 1 H; J = 2.8 Hz); 7.27 (bs, 1 H); 7.03 (s, 1 H); 5.01 (s, 2H); 4.00 (s, 2H); 2.70 (bs, 1H); 2.56 (bs, 1H);1.80-1.13 (m, 8H); 0.74 (d, 3H; J = 1.6 Hz). ES-MS: 317 [M+H]⁺

### 4-(2,4-dichlorophenyl)-3-phenethyl-3,4-dihydro-2H-[1,3]oxazino[5,6-h]quinoline (SST0824AA1): amine = phenylethylamine, aldehyde = 2,4-dichlorobenzaldehyde

¹H NMR (400 MHz, CDCl₃, δ ppm) δ 8.93 (d, 1 H; *J* = 4.2 Hz), 8.07 (d, 2H; *J* = 8.3 Hz), 7.49 - 7.37 (m, 2H), 7.32-7.20 (m, 2H); 7.13 - 7.00 (m, 2H), 6.95 (d, 1 H; *J* = 8.4 Hz), 6.75 (d, 1 H; *J* = 8.3 Hz), 5.09 (d, 2H; *J =* 10.7 Hz), 4.89 (d, 2H; *J =* 10.7 Hz), 3.43 - 3.31 (m, 2H), 3.15-3.05 (m, 2H). ¹³C-NMR (100 MHz, CDCl₃, δ ppm) 150.03, 149.24, 139.07, 138.48, 135.57, 135.22, 133.79, 131.78, 129.52, 128.31, 128.21, 127.90, 126.78, 126.03, 125.68, 121.38, 118.64, 117.08, 59.69, 54.27, 34.38.

### Example 5

### Biological activity of Gli inhibitors

### Example 5.1 - Effect on Gli1 protein levels

### Materials and methods

The effect of compounds on Gli1 protein expression was evaluated in HH-responsive murine NIH3T3 cells by Western blotting. Both Gli1 and Ptch1 are targets of the Gli, and Gli1 in particular is considered the best read-out of HH signaling activity.

Briefly, NIH3T3 were stimulated for 48 h with 3-chloro-N-[trans-4-(methylamino)cyclohexyl]-N-[[3-(4-pyridinyl)phenyl]methyl]-benzo[*b*]thiophene-2-carboxamide (SAG) (100nM) to activate the endogenous HH pathway, and treated for 24 or 48 h with each compound (1 µM). NIH3T3 cells were lysed and subjected to Western blotting using a specific anti-GLI1 antibody. The effect of each compound was compared to that of GANT61 (5 µM), a well known Gli1/2 inhibitor (Lauth M. et al. PNAS 2007, 104, 8455).

### Results

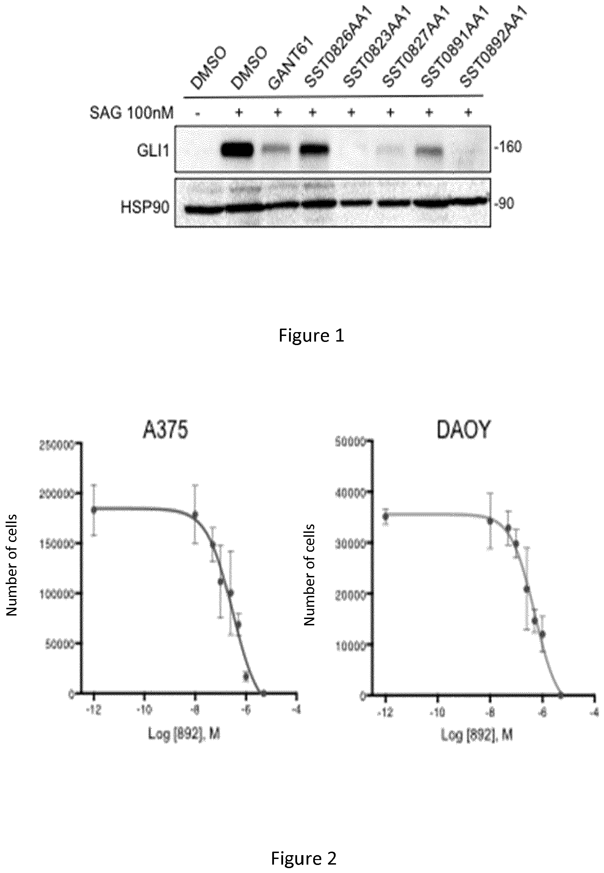

The results are reported in Figure 1.

Note that Gli1 is activated upon SAG administration.

Treatment with SST0823AA1, SST0827AA1 and SST0892AA1 led to a drastic reduction of Gli1 protein level, suggesting a strong inhibition of the HH pathway. SST0891AA1 produced an effect on Gli1 reduction comparable to that obtained with GANT61, used as a reference compound. SST0826AA1 induces a slight reduction in Gli1 protein level.

HSP90 was used as a loading control.

### Example 5.2 - Effects on cancer cell proliferation

The effects on cancer cell proliferation were tested for those compounds shown to be able to reduce Gli1 protein level in NIH3T3 cells. Two different commercial cancer cell lines were used: A375 (metastatic melanoma cell line) and DAOY (medulloblastoma cell line).

### Materials and methods

To test the effects on cancer cell proliferation, both cell types were treated for 72 h with increasing doses (10 nM, 50 nM, 100 nM, 250 nM, 500 nM, 1 µM, 5 µM) of each compound and counted at the end of the treatment with an MTT-based assay. IC₅₀ was calculated by GraphPad Prism software.

### Results

The effect on different cancer cell line proliferation is summarized in the following tables 2 and 3 for some of the most representative active compounds.

In particular, Table 2 shows results for newly obtained compounds, table 3 shows results of commercially available compounds.

**Table 2: Summary of IC₅₀ and effects on Gli1 protein for selected new compounds. Legend:**

| Compound | Effect on Gli1 protein | Effect on proliferation A375 (IC₅₀; nM) | Effect on proliferation DAOY (IC₅₀; nM) |
|---|---|---|---|
| SST0823AA1 | +++ | +++ | +++ |
| SST0891AA1 | + | + | + |
| SST0892AA1 | +++ | +++ | ++ |
| SST0893AA1 | + | ++ | ++ |
| SST0910AA1 | + | ++ | ++ |
| SST0912AA1 | + | ++ | + |
| SST0913AA1 | + | ++ | + |
| SST0827AA1 | +++ | +++ | +++ |

### Effect on Gli1 protein:

- +++:: Gli1 protein level strongly reduced (almost abolished) at 24 and 48 h compared to DMSO control. A strong reduction already at 24 h indicates that the effect of the compound on Gli1 is fast.
- ++:: Gli1 protein level reduced (more than 50%) at 48 h compared to DMSO control.
- +:: Gli1 protein level is weakly reduced (about 50%) at 48 h compared to DMSO control.

### Effect on cancer cell proliferation:

- +:: IC₅₀ ≥ 1000 nM
- ++:: IC₅₀ between 300 nM and 1000 nM
- +++:: IC₅₀ ≤ 300 nM

**Table 3: Summary of the effects of commercially available Compounds on A375 and DAOY cell proliferation and Gli1 protein.**

| Compound | Effect on Gli1 protein | Effect on proliferation A375 (IC₅₀; nM) | Effect on proliferation DAOY (IC₅₀; nM) |
|---|---|---|---|
| SST0687AA1 | +++ | ++ | ++ |
| SST0689AA1 | +++ | ++ | ++ |
| SST0769AA1 | ++ | ++ | ++ |
| SST0771AA1 | +++ | ++ | ++ |
| SST0773AA1 | + | ++ | ++ |
| SST0774AA1 | +++ | +++ | +++ |
| SST0775AA1 | + | ++ | ++ |
| SST0776AA1 | +++ | +++ | +++ |
| SST0777AA1 | +++ | ++ | ++ |
| SST0791AA1 | ++ | +++ | +++ |
| SST0792AA1 | + | +++ | + |
| SST0793AA1 | ++ | ++ | ++ |
| SST0794AA1 | ++ | +++ | +++ |

### Legend:

### Effect on Gli1 protein:

- +++:: Gli1 protein level strongly reduced (almost abolished) at 24 and 48 h compared to DMSO control. A strong reduction already at 24 h indicates that the effect of the compound on Gli1 is fast.
- ++:: Gli1 protein level reduced (more than 50%) at 48 h compared to DMSO control.
- +:: Gli1 protein level is weakly reduced (about 50%) at 48 h compared to DMSO control.

### Effect on cancer cell proliferation:

- +:: IC₅₀ ≥ 1000 nM
- ++:: IC₅₀ between 300 nM and 1000 nM
- +++:: IC₅₀ ≤ 300 nM

The compounds of the invention were selected based on their ability to drastically reduce Gli1 protein level and at the same time to diminish cell proliferation *in vitro* in two independent cell lines (A375 and DAOY) (Tables 2 and 3).

As summarised in Table 2, the best compounds appear to be SST0823AA1, SST0827AA1 and SST0892AA1. All three these compounds are able to abolish Gli1 protein level (Figure 1) and to be effective at reducing cancer cell proliferation at nM doses, suggesting that the reduction in cancer cell proliferation depends on selective inhibition of Gli1.

Figure 2 shows dose-response curves in A375 and DAOY cells treated with increasing doses of SST0892AA1 for 72 h. Graphs were obtained with GraphPad Prism. IC₅₀ is 300 nM for A375 and 500 nM for DAOY.

## Claims

1. A compound **characterized by** the following formula (I) 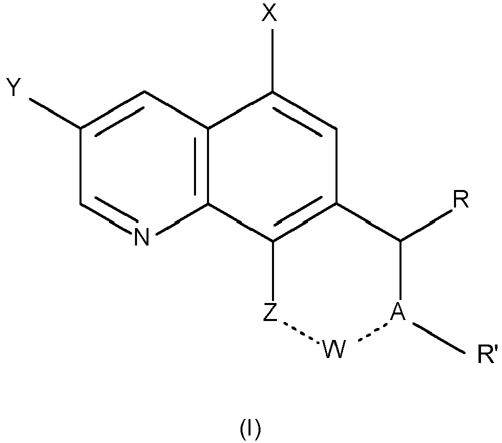 wherein
A is selected from N and NH,
X is selected from H and a halogen;
Y is selected from the group consisting of H, halogen, O-C₁-C₈alkyl, linear or branched, O-C₅-C₁₂aryl, O-C₁-C₈acyl, linear or branched, and O-C₃-C₈cycloalkyl;
Z is selected from O and OH;
W is CH₂ or CO or is absent and when W is CH₂ or CO each dashed line indicates a single bond,
R and R' are independently selected from the group consisting of: H; C₁-C₈alkyl, linear or branched; C₁-C₈acyl, linear or branched; C₃-C₈ cycloalkyl or heterocycloalkyl; C₅-C₁₄aryl or heteroaryl; CH(COOCH₃)(CH₂phenyl) and C₁-C₈alkylene-R²,
wherein R² is selected from the group consisting of H, C₁-C₈alkyl, linear or branched, C₁-C₈acyl, linear or branched, C₃-C₈cycloalkyl or heterocycloalkyl, C₅-C₁₄aryl or heteroaryl,
said heterocycloalkyl and heteroaryl containing one or more heteroatoms selected from N, S and O in a monocyclic or polycyclic system,
said cycloalkyl, heterocycloalkyl, aryl or heteroaryl being optionally substituted on any position of the ring with one or more substituents selected from the group consisting of halogen, C₁-C₄alkyl, linear or branched, O-C₁-C₄alkyl, linear or branched, C₁-C₈acyl, linear or branched, an amino group optionally substituted with one or two C₁-C₄alkyl groups, SH and OH,
the enantiomers, diastereoisomers, and mixtures thereof,
or a pharmaceutically acceptable salt, hydrate or solvate thereof,
for use in the treatment of a disease which can be improved or prevented by the inhibition of the Gli1 protein.

2. The compound for the use of claim 1 wherein said disease is a tumor or metastasis.

3. The compound for the use of claim 2 wherein said tumor is a Hedgehog dependent tumor.

4. The compound for the use of claim 2 or 3 wherein said tumor is selected from the group consisting of basal cell carcinoma, medulloblastoma, skin tumor, brain tumor, lungs tumor, prostate tumor, breast tumor, gastrointestinal tract tumor, blood tumors, and melanoma.

5. The compound for the use of anyone of the preceding claims wherein said compound of formula (I) is used for killing cancer stem cells and/or cancer progenitor cells.

6. The compound for the use of anyone of claims 1-5 wherein R is a phenyl.

7. The compound for the use of anyone of claims 1-6 wherein R' is an acyl group.

8. The compound for the use of anyone of claims 1-6 wherein R' is C₁-C₈alkylene-R², wherein R² is selected from an aryl, preferably a phenyl or an indole, and a cycloalkyl, preferably a cyclohexyl.

9. The compound for the use according to anyone of the preceding claims, which is one of the followings:
| Compound name | Compound structural formula |
|---|---|
| Pentanamide, N-[(5-chloro-8-hydroxy-7-quinolinyl)[4-(1-methylethyl)phenyl]methyl] | 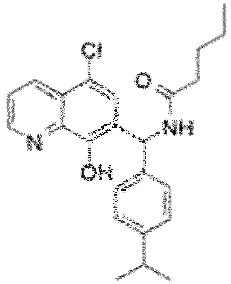 |
| N-[(5-Chloro-8-hydroxy-quinolin-7-yl)-(3,4-dimethoxy-phenyl)-methyl]-acetamide | 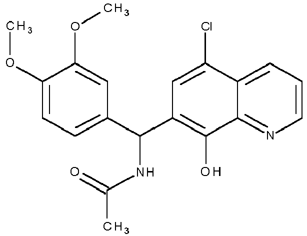 |
| N-[(5-Chloro-8-hydroxy-quinolin-7-yl)-(2,4-dichloro-phenyl)-methyl]-butyramide | 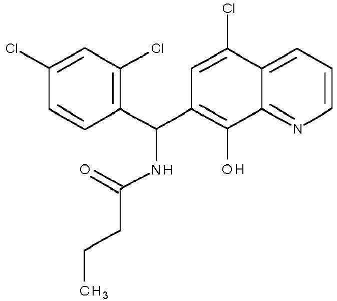 |
| Propanamide, N-[(5-chloro-8-hydroxy-7-quinolinyl)(3,4-dimethoxyphenyl)methyl]-2-methyl- | 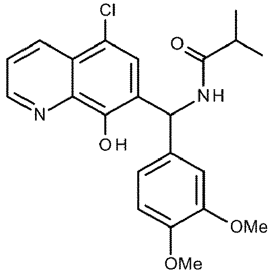 |
| Butanamide, N-[(5-chloro-8-hydroxy-7-quinolinyl)(4-chlorophenyl)methyl]- | 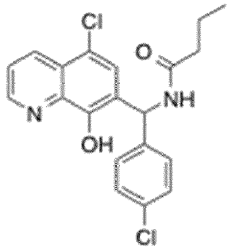 |
| Butanamide, N-[(5-chloro-8-hydroxy-7-quinolinyl)[4-(diethylamino)phenyl]methyl]- | 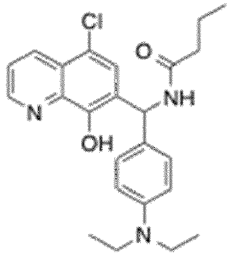 |
| Butanamide, N-[(5-chloro-8-hydroxy-7-quinolinyl)phenylmethyl]- | 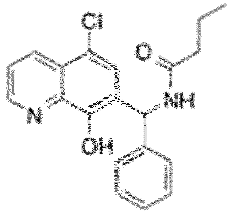 |
| Butanamide, N-[(5-chloro-8-hydroxy-7-quinolinyl)(4-methylphenyl)methyl]- | 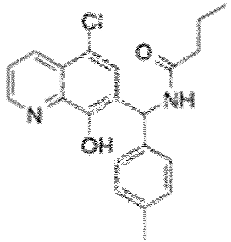 |
| Butanamide, N-[(5-chloro-8-hydroxy-7-quinolinyl)(2-chlorophenyl)methyl]- | 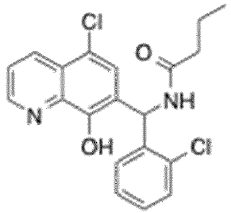 |
| Butanamide, N-[(2,4-dichlorophenyl)(8-hydroxy-7-quinolinyl)methyl]- | 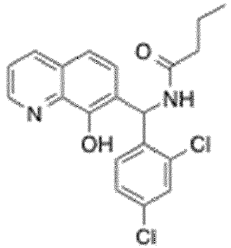 |
| Pentanamide, N-[(3,4-dimethoxyphenyl) (8-hydroxy-7-quinolinyl)methyl]- | 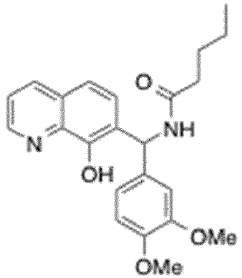 |
| 2H-Pyrido[3,2-h]-1,3-benzoxazine, 6-chloro-3-[2-(1-cyclohexen-1-yl)ethyl]-3,4-dihydro- | 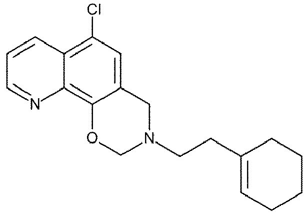 |
| 2H-Pyrido[3,2-h]-1,3-benzoxazine, 6-chloro-3,4-dihydro-3-[3-(4-morpholinyl) propyl]- | 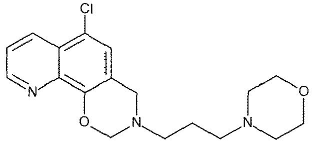 |
| 2H-Pyrido[3,2-h]-1,3-benzoxazine, 3-butyl-6-chloro-3,4-dihydro- | 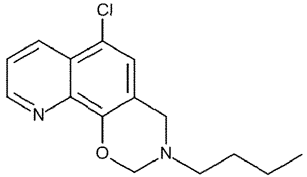 |
| 2H-Pyrido[3,2-h]-1,3-benzoxazine, 6-chloro-3,4-dihydro-3-(2-phenylethyl)- | 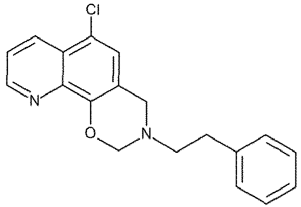 |

10. The compound for the use according to anyone of claims 1-8, which is one of the followings:
| | |
|---|---|
| 3-phenethyl-3,4-dihydro-2H-[1,3]oxazino[5,6-h]quinoline | 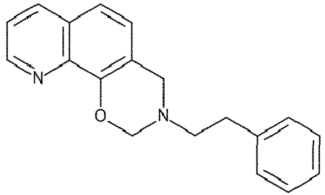 |
| 4-(2,4-dichlorophenyl)-3-phenethyl-3,4-dihydro-2H-[1,3]oxazino[5,6-h]quinoline | 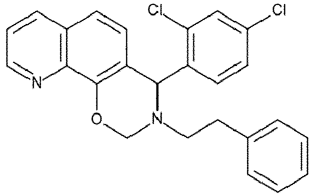 |
| 3-phenethyl-3,4-dihydro-2H-[1,3]oxazino[5,6-h]quinolin-2-one | 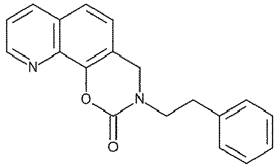 |
| 4-(2,4-dichlorophenyl)-3-phenethyl-3,4-dihydro-2H-[1,3]oxazino[5,6-h]quinolin-2-one | 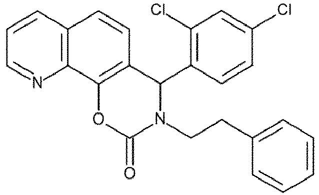 |
| 7-((2,4-dichlorophenyl)(phenethylamino)methyl)q uinolin-8-ol | 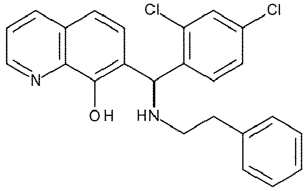 |
| 3-(2-(1H-indol-3-yl)ethyl)-6-chloro-3,4-dihydro-2H-[1,3]oxazino[5,6-h]quinoline | 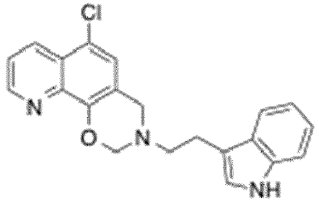 |
| 5-chloro-7-[(phenethylamino)methyl]quinolin-8-ol | 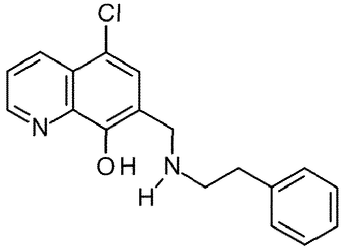 |
| 3-(2-(1H-indol-3-yl)ethyl)-3,4-dihydro-2H-[1,3]oxazino[5,6-h]quinoline | 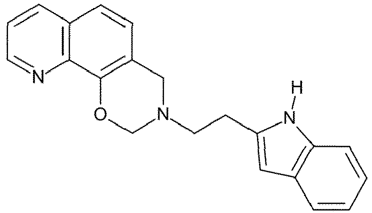 |
| 8-fluoro-3-phenethyl-3,4-dihydro-2H-[1,3]oxazino[5,6-h]quinoline | 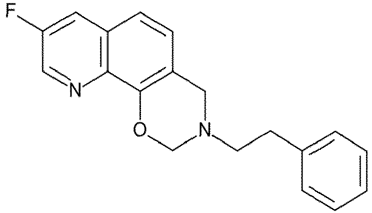 |
| 3-cyclohexyl-3,4-dihydro-2H-[1,3]oxazino[5,6-h]quinoline | 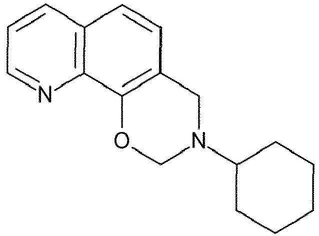 |
| 3-(1,2,3,4-tetrahydronaphthalen-2-yl)-3,4-dihydro-2H-[1,3]oxazino[5,6-h]quinoline | 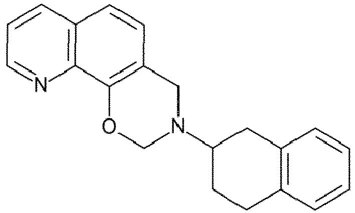 |
| 3-(4-methoxybenzyl)-3,4-dihydro-2H-[1,3]oxazino[5,6-h]quinoline | 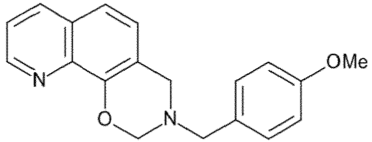 |
| Methyl (S)-2-(2H-[1,3]oxazino[5,6-h]quinolin-3(4H)-yl)-3-phenylpropanoate | 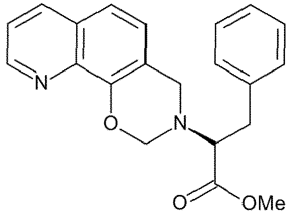 |
| 3-(cyclohexylmethyl)-3,4-dihydro-2H-[1,3]oxazino[5,6-h]quinoline | 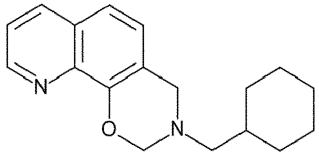 |
| 3-cycloheptyl-3,4-dihydro-2H-[1,3]oxazino[5,6-h]quinoline | 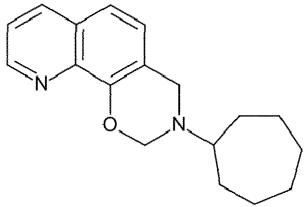 |
| 3-cyclohexyl-8-fluoro-3,4-dihydro-2H-[1,3]oxazino[5,6-h]quinoline | 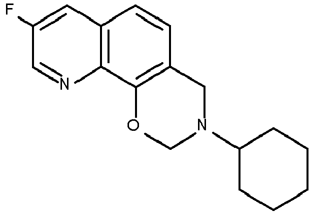 |
| 6-chloro-3-cycloheptyl-3,4-dihydro-2H-[1,3]oxazino[5,6-h]quinoline | 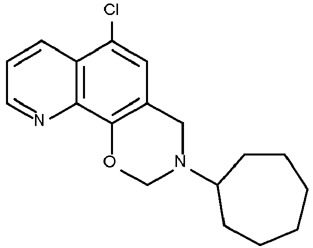 |
| 6-chloro-3-(4-methylcyclohexyl)-3,4-dihydro-2H-[1,3]oxazino[5,6-h]quinoline | 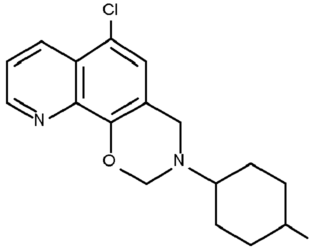 |

11. A pharmaceutical composition containing at least one compound of formula (I) according to anyone of claims 1-10 as an active ingredient together with at least one pharmaceutically acceptable vehicle and/or excipient for use in the treatment of a disease which can be improved or prevented by the inhibition of the Gli1 protein.

12. The pharmaceutical composition for the use according to claim 11 further comprising a chemotherapeutic agent.

13. A compound having the following formula (IIa) 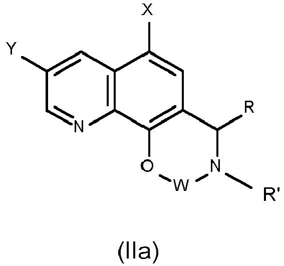 wherein
X is selected from H and halogen;
Y is selected from the group consisting of H, halogen, O-C₁-C₈alkyl, linear or branched, O-C₅-C₁₂aryl, O-C₁-C₈acyl, linear or branched, and O-C₃-C₈cycloalkyl;
W is selected from CH₂ and CO,
R is selected from H and C₆aryl,
R' is selected from the group consisting of: H; C₁-C₈alkyl, linear or branched; C₁-C₈acyl, linear or branched; C₃-C₈ cycloalkyl or heterocycloalkyl; C₅-C₁₄aryl or heteroaryl; CH(COOCH₃)(CH₂phenyl) and C₁-C₈alkylene-R²,
wherein R² is selected from the group consisting of H, C₁-C₈alkyl, linear or branched, C₁-C₈acyl, linear or branched, C₃-C₈cycloalkyl or heterocycloalkyl, C₅-C₁₀aryl or heteroaryl,
said heterocycloalkyl and heteroaryl containing one or more heteroatoms selected from N, S and O in a monocyclic or polycyclic system,
said cycloalkyl, heterocycloalkyl, aryl or heteroaryl being optionally substituted on any position of the ring with one or more substituents selected from the group consisting of halogen, C₁-C₄alkyl, linear or branched, O-C₁-C₄alkyl, linear or branched, an amino group optionally substituted with one or two C₁-C₄alkyl groups, SH and OH,
wherein
when W is CH₂, R is H and X is halogen, R' is selected from the group consisting of C₁-C₈alkylene-indolyl and totally saturated cycloalkyl, optionally substituted,
the enantiomers, diastereoisomers, and mixtures thereof,
or a pharmaceutically acceptable salt, hydrate or solvate thereof.

14. The compound according to claim 13 wherein W is CH₂, R is H and X is H.

15. The compound according to claim 14 wherein R' is selected from cycloalkyl and C₁-C₈alkylene-R² wherein R² is preferably phenyl.

16. The compound according to anyone of claims 13-15 which is one of the followings:
3-phenethyl-3,4-dihydro-2*H*-[1,3]oxazino[5,6-*h*]quinoline,
4-(2,4-dichlorophenyl)-3-phenethyl-3,4-dihydro-2*H*-[1,3]oxazino[5,6-*h*]quinoline
3-phenethyl-3,4-dihydro-2*H*-[1,3]oxazino[5,6-*h*]quinolin-2-one
4-(2,4-dichlorophenyl)-3-phenethyl-3,4-dihydro-2*H-*[1,3]oxazino[5,6-*h*]quinolin-2-one
3-(2-(1*H*-indol-3-yl)ethyl)-6-chloro-3,4-dihydro-2*H*-[1,3]oxazino[5,6-*h*]quinoline
3-(2-(1*H*-indol-3-yl)ethyl)-3,4-dihydro-2H-[1,3]oxazino[5,6-*h*]quinoline
8-fluoro-3-phenethyl-3,4-dihydro-2*H*-[1,3]oxazino[5,6-*h*]quinoline
3-cyclohexyl-3,4-dihydro-2*H*-[1,3]oxazino[5,6-*h*]quinoline
3-(1,2,3,4-tetrahydronaphthalen-2-yl)-3,4-dihydro-2*H*-[1,3]oxazino[5,6-*h*]quinoline
3-(4-methoxybenzyl)-3,4-dihydro-2*H*-[1,3]oxazino[5,6-*h*]quinoline
Methyl (S)-2-(2*H*-[1,3]oxazino[5,6-*h*]quinolin-3(4*H*)-yl)-3-phenylpropanoate
3-(cyclohexylmethyl)-3,4-dihydro-2*H*-[1,3]oxazino[5,6-*h*]quinoline
3-cycloheptyl-3,4-dihydro-2*H*-[1,3]oxazino[5,6-*h*]quinoline
3-cyclohexyl-8-fluoro-3,4-dihydro-2*H*-[1,3]oxazino[5,6-*h*]quinoline
6-chloro-3-cycloheptyl-3,4-dihydro-2*H*-[1,3]oxazino[5,6-*h*]quinoline and
6-chloro-3-(4-methylcyclohexyl)-3,4-dihydro-2*H*-[1,3]oxazino[5,6-*h*]quinoline.

17. A compound having the following formula (IIb) 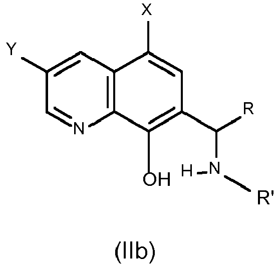 wherein
X is selected from H and halogen;
Y is selected from the group consisting of H, halogen, O-C₁-C₈alkyl, linear or branched, O-C₅-C₁₂aryl, O-C₁C₈acyl, linear or branched, and O-C₃-C₈cycloalkyl,
R is selected from H and C₆aryl;
R' is selected from the group consisting of: H; C₁-C₈alkyl, linear or branched; C₃-C₈ cycloalkyl or heterocycloalkyl; C₅-C₁₄aryl or heteroaryl; CH(COOCH₃)(CH₂phenyl) and C₁-C₈alkylene-R²,
wherein R² is selected from the group consisting of H, C₁-C₈alkyl, linear or branched, C₃-C₈cycloalkyl or heterocycloalkyl, C₅-C₁₀aryl or heteroaryl,
said heterocycloalkyl and heteroaryl containing one or more heteroatoms selected from N, S and O in a monocyclic or polycyclic system,
said cycloalkyl, heterocycloalkyl, aryl or heteroaryl being optionally substituted on any position of the ring with one or more substituents selected from the group consisting of halogen, C₁-C₄alkyl, linear or branched, O-C₁-C₄alkyl, linear or branched, an amino group optionally substituted with one or two C₁-C₄alkyl groups, SH and OH,
wherein
when X is H, R' is selected from the group consisting of totally saturated cycloalkyl, optionally substituted, and C₁-C₈alkylene-R²,
the enantiomers, diastereoisomers, and mixtures thereof,
or a pharmaceutically acceptable salt, hydrate or solvate thereof.

18. The compound according to claim 17 wherein X is H, R is a phenyl, preferably a dichlorophenyl, and R' is C₁-C₈alkylene-R², R² being preferably a phenyl.

19. The compound according to anyone of claims 17-18 which is one of the followings:
7-((2,4-dichlorophenyl)(phenethylamino)methyl)quinolin-8-ol,
5-chloro-7-[(phenethylamino)methyl]quinolin-8-ol.

20. The compound of anyone of claims 13-19 for use as a medicament.

21. A pharmaceutical composition containing as active ingredient the compound of anyone of claims 13-19 and at least one pharmaceutically acceptable vehicle and/or excipient.

22. The pharmaceutical composition according to claim 21 wherein said composition further comprises a chemotherapeutic agent.

23. The pharmaceutical composition according to claim 22 wherein said chemotherapeutic agent is selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens.

24. The pharmaceutical composition according to claim 23 wherein said chemotherapeutic agent is a cytotoxic agent selected from the group consisting of melphalan, irinotecan, rituximab and lapatinib; or is a proteasome inhibitor selected from the group consisting of bortezomib, carfilzomib and ixazomib; or is an immunomodulatory agent selected from the group consisting of thalidomide, lenalidomide and pomalidomide.
